# EUROPEAN PATENT SPECIFICATION

(11) **EP 4 034 875 B1**
(45) Date of publication and mention of the grant of the patent: **30.10.2024**
(21) Application number: 20780609.2
(22) Date of filing: 22.09.2020
(51) Int. Cl.: G01N 33/487

(54) **INTERFACE FOR AUTOMATED FLUID INJECTION**
SCHNITTSTELLE FÜR AUTOMATISIERTE FLÜSSIGKEITSEINSPRITZUNG
INTERFACE POUR INJECTION AUTOMATISÉE DE FLUIDE

(30) Priority: 25.09.2019 US 201962905972 P
(43) Date of publication of application: 03.08.2022
(73) Proprietor: F. Hoffmann-La Roche AG, 4070 Basel (CH); Roche Diagnostics GmbH, 68305 Mannheim (DE)
(72) Inventor: DIPIETRO, Matthew, Pleasonton, California 94588 (US); ENGELBART, Damion, Pleasonton, California 94588 (US); LIU, Andrew, Pleasonton, California 94588 (US); NIELSEN, William, Pleasonton, California 94588 (US); WOJTOWICZ, Janusz B., Pleasonton, California 94588 (US); YUAN, Robert A., Pleasonton, California 94588 (US)
(74) Representative: Merkel, Patrick
(86) International application number: PCT/EP2020/076372
(87) International publication number: WO 2021/058456

(56) References cited:
- EP-A1- 2 623 960
- JP-A- 2011 099 867
- US-A1- 2010 133 510
- US-A1- 2016 274 082
- US-A1- 2017 212 078

## Description

### FIELD

Embodiments of the invention relate generally to systems and methods for sequencing, and more particularly to a consumable device that can be used with a sequencing system and method.

### BACKGROUND OF THE INVENTION

Advances in micro-miniaturization within the semiconductor industry in recent years have enabled biotechnologists to begin packing traditionally bulky sensing tools into smaller and smaller form factors, onto so-called biochips. It would be desirable to develop techniques for biochips that make them more robust, efficient, and cost-effective. US 2016/274082 A1, US 2017/212078 A1, EP 2 623 960 A1, JP 2011 099867 and US 2010/133510 disclose consumable devices similar where the connection to a dispensing device is not satisfying.

### SUMMARY OF THE DISCLOSURE

The present invention relates to a consumable device for use with a sequencing system according to claim 1 and a method of delivering fluid to a consumable device for sequencing according to claim 14.

In some embodiments, a consumable device for use with a sequencing system is provided. The consumable device can include a sequencing chip, the sequencing chip can include a plurality of wells, each well including a working electrode; a flow cell including at least one flow channel, wherein the flow cell is configured to be disposed over sequencing chip such that the at least one flow channel is disposed over the plurality of wells of the sequencing chip; at least one inlet boss having a lumen in fluid communication with the at least one flow channel; and at least one counter electrode disposed over at least a portion of the at least one flow channel; and a flow cell cover including at least one inlet boss receptacle for receiving the at least one inlet boss of the flow cell; and at least one dispense tip receptacle configured to receive a dispense tip, wherein the at least one dispense tip receptacle is in fluid communication the at least one inlet boss receptacle.

In some embodiments, the sequencing chip includes at least about 1, 2, 3, 4, 5, 6, 7, or 8 million wells.

In some embodiments, the at least one dispense tip receptacle is funnel shaped.

In some embodiments, the at least one dispense tip receptacle is connected to the at least one inlet boss receptacle via a hole or lumen sized to pass the dispense tip.

In some embodiments, the lumen of the at least one inlet boss has a constant diameter.

In some embodiments, the lumen of the at least one inlet boss is tapered.

In some embodiments, the lumen of the at least one inlet boss includes a chamfered inlet opening.

In some embodiments, the at least one inlet boss is made of a deformable material capable of conforming to the dispense tip.

In some embodiments, the at least one inlet boss receptacle has a bowed surface configured to form a gap with the at least one inlet boss when the at least one inlet boss is inserted into the at least one inlet boss receptacle.

In some embodiments, the at least one inlet boss receptacle has a chamfered opening.

In some embodiments, the at least one inlet boss comprises a plurality of inlet lumens that are combined into a single inlet boss.

In some embodiments, each inlet lumen has a conical opening, wherein the at least one dispense tip receptacles includes a plurality of dispense tip receptacles in fluid communication with a single inlet boss receptacle configured to receive the single inlet boss, wherein the single inlet boss receptacle comprises a plurality of conical sealing surfaces configured to mate with each conical opening of each inlet lumen.

In some embodiments, the at least one dispense tip receptacle is sealed with a pierceable material.

In some embodiments, a method of delivering fluid to a consumable device for sequencing is provided. The method can include inserting a piercing tool through a seal that is covering a dispense tip receptacle of a consumable device. The consumable device can include a sequencing chip, the sequencing chip including a plurality of wells, each well including a working electrode; a flow cell including at least one flow channel, wherein the flow cell is configured to be disposed over sequencing chip such that the at least one flow channel is disposed over the plurality of wells of the sequencing chip; at least one inlet boss having a lumen in fluid communication with the at least one flow channel; and at least one counter electrode disposed over at least a portion of the at least one flow channel; and a flow cell cover including at least one inlet boss receptacle for receiving the at least one inlet boss of the flow cell; and at least one dispense tip receptacle configured to receive a dispense tip, wherein the at least one dispense tip receptacle is in fluid communication the at least one inlet boss receptacle. The method can further include removing the piercing tool from the dispense tip receptacle and leaving one or more seal fragments that extend into the dispense tip receptacle; inserting a distal end of a dispense tip past the one or more seal fragments; after the step of inserting the distal end of the dispense tip past the one or more seal fragments, partially dispensing a first fluid from the distal end of the dispense tip so that the first fluid extends distally from the distal end as a partial droplet; and advancing the dispense tip so that the partial droplet makes a liquid-liquid connection with a second fluid in the dispense tip receptacle.

In some embodiments, the method further includes advancing the distal end of the dispense tip into the lumen of the at least one inlet boss receptacle to make a fluid tight seal between the dispense tip and the at least one inlet boss.

In some embodiments, the method further includes delivering fluid through the dispense tip into the at least one flow channel.

In some embodiments, the method further includes piercing a seal of a reagent reservoir with the piercing tool, wherein the first fluid is stored in the reagent reservoir.

In some embodiments, the seal is a pierceable cap.

In some embodiments, the reagent reservoir is selected from the group consisting of a bottle and a trough.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Figure 1 illustrates an embodiment of a cell 100 in a nanopore based sequencing chip.
Figure 2 illustrates an embodiment of a cell 200 performing nucleotide sequencing with the Nano-SBS technique.
Figure 3 illustrates an embodiment of a cell about to perform nucleotide sequencing with pre-loaded tags.
Figure 4 illustrates an embodiment of a process 400 for nucleic acid sequencing with pre-loaded tags.
Figure 5 illustrates an embodiment of a fluidic workflow process 500 for flowing different types of liquids or gases through the cells of a nanopore based sequencing chip during different phases of the chip's operation.
Figure 6A illustrates an exemplary flow of a liquid or gas across the nanopore based sequencing chip.
Figure 6B illustrates another exemplary flow of a liquid or gas across the nanopore based sequencing chip.
Figure 7A illustrates an exemplary flow of a first type of fluid across the nanopore based sequencing chip.
Figure 7B illustrates that a second fluid is flowed through the chip after a first fluid has been flowed through the chip at an earlier time.
Figure 8 illustrates the top view of a nanopore based sequencing system 800 with a flow chamber enclosing a silicon chip that allows liquids and gases to pass over and contact sensors on the chip surface.
Figure 9 illustrates the various components that are assembled together to form the nanopore based sequencing system 800 as shown in Figure 8.
Figure 10 illustrates another exemplary view of nanopore based sequencing system 800.
Figure 11A illustrates the top view of a nanopore based sequencing system 1100 with an improved flow chamber enclosing a silicon chip that allows liquids and gases to pass over and contact sensors on the chip surface.
Figure 11B illustrates the cross sectional view of system 1100 from the position of a plane 1114 through the system.
Figure 12A illustrates another exemplary view of nanopore based sequencing system 1100 with a fan-out plenum.
Figure 12B illustrates the various components that are assembled together to form nanopore based sequencing system 1100 as shown in Figure 11.
Figure 13 illustrates the paths that are followed by a fluid as it flows through the nanopore based sequencing system 1100 with a fan-out plenum.
Figure 14 illustrates the top view of a nanopore based sequencing system 1400 with another improved flow chamber enclosing a silicon chip that allows liquids and gases to pass over and contact sensors on the chip surface.
Figure 15 illustrates the top view of a nanopore based sequencing system 1500 with another improved flow chamber enclosing a silicon chip that allows liquids and gases to pass over and contact sensors on the chip surface.
Figure 16 illustrates the top view of a nanopore based sequencing system 1600 with another improved flow chamber enclosing a silicon chip that allows liquids and gases to pass over and contact sensors on the chip surface.
Figure 17 illustrates the top view of a nanopore based sequencing system 1700 with another improved flow chamber enclosing a silicon chip that allows liquids and gases to pass over and contact sensors on the chip surface.
Figure 18 illustrates the top view of a nanopore based sequencing system 1800 with another improved flow chamber enclosing a silicon chip that allows liquids and gases to pass over and contact sensors on the chip surface.
Figure 19A illustrates an exemplary view of one embodiment of a nanopore based sequencing system 1900 with a serpentine flow channel.
Figure 19B illustrates the various components that are laminated together to form nanopore based sequencing system 1900.
Figure 20A illustrates the top side view of a backing plate and a flexible flat circuit that is connected to the counter electrode (not visible) located on the bottom side of the backing plate.
Figure 20B illustrates the same unit 2000 as shown in Figure 20A when the backing plate is flipped upside down.
Figure 20C illustrates the various components of unit 2000 that are laminated together.
Figure 21A illustrates a cross sectional view of a flow channel 2100 with sharp edges or sharp corners that may trap fluids more easily.
Figure 21B illustrates a cross sectional view of a flow channel 2102 that has a D-shaped cross sectional geometry.
Figure 21C illustrates a cross sectional view of another flow channel 2106 that has a D-shaped cross sectional geometry.
Figure 22 illustrates a side view of a nanopore based sequencing system with flow channels having a D-shaped cross sectional geometry.
Figure 23 is a diagram illustrating an embodiment of a molded flow channel component.
Figure 24 is a diagram illustrating an embodiment of a counter electrode insert.
Figure 25 is a diagram illustrating an embodiment of a mold for a molded flow channel component.
Figure 26 is a diagram illustrating an embodiment of a molded flow channel component removed from a mold.
Figure 27 is a diagram illustrating a portion of an embodiment of a nanopore based sequencing system utilizing a molded flow channel component.
Figure 28 is a diagram illustrating an embodiment of a clamping of nanopore based sequencing system components.
Figure 29 is a diagram illustrating an embodiment of encapsulated wire bonds.
Figure 30 is a diagram illustrating an embodiment of encapsulating wire bonds together with one or more flow channel components in a single manufacturing step.
Figures 31A and 31B illustrate an embodiment of an adapter that helps form a fluidic seal between the flow cell and the dispense tip.
Figures 32A-32L illustrate various embodiments of fluidic interfaces that can be integrated into the flow cell.
Figures 33A-33C illustrate additional embodiments of fluidic interfaces that can be integrated into the flow cell.
Figures 34A-34E illustrate an embodiment of a dispense tip that can be inserted into the fluidic interface of the flow cell to form a fluidic seal.
Figures 35A and 35B illustrate another embodiment of a dispense tip.
Figures 36A-36F illustrate and embodiment of a piercing tool that can be used to create an opening in a seal covering the fluidic interface.
Figures 37A and 37B illustrate the insertion of a piercing tool into a receptacle of a flow cell.
Figures 38A-38C illustrate the insertion of the piercing tool shown in Figures 37A and 37B through a reagent bottle cap.
Figures 39A-39E illustrate a method of inserting the dispense tip into the fluidic interface in a manner that does not introduce air bubbles into the flow cell.

### DETAILED DESCRIPTION

Unless stated otherwise, a component such as a processor or a memory described as being configured to perform a task may be implemented as a general component that is temporarily configured to perform the task at a given time or a specific component that is manufactured to perform the task. As used herein, the term 'processor' refers to one or more devices, circuits, and/or processing cores configured to process data, such as computer program instructions.

A detailed description of one or more embodiments of the invention is provided below along with accompanying figures that illustrate the principles of the invention. The invention is described in connection with such embodiments, but the invention is not limited to any embodiment. The scope of the invention is limited only by the claims . Numerous specific details are set forth in the following description in order to provide a thorough understanding of the invention. These details are provided for the purpose of example and the invention may be practiced according to the claims without some or all of these specific details. For the purpose of clarity, technical material that is known in the technical fields related to the invention has not been described in detail so that the invention is not unnecessarily obscured.

Nanopore membrane devices having pore sizes on the order of one nanometer in internal diameter have shown promise in rapid nucleotide sequencing. When a voltage potential is applied across a nanopore immersed in a conducting fluid, a small ion current attributed to the conduction of ions across the nanopore can be observed. The size of the current is sensitive to the pore size.

A nanopore based sequencing chip may be used for DNA sequencing. A nanopore based sequencing chip incorporates a large number of sensor cells configured as an array. For example, an array of one million cells may include 1000 rows by 1000 columns of cells.

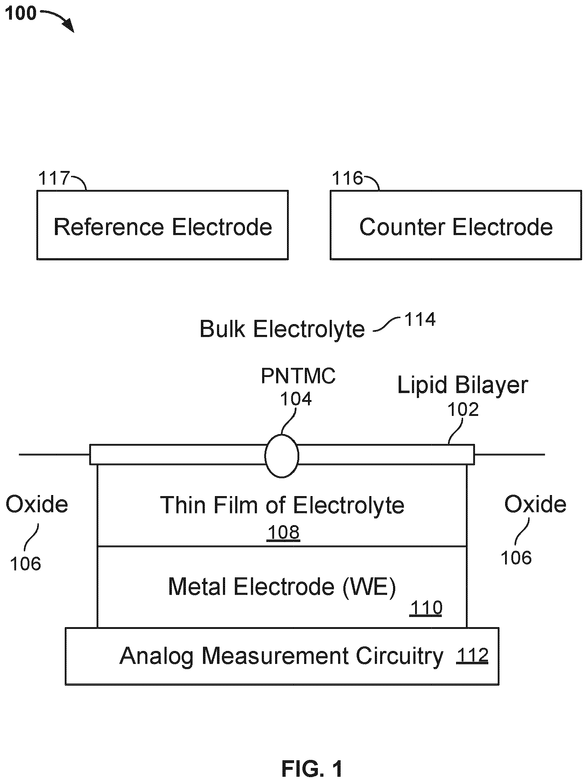

Figure 1 illustrates an embodiment of a cell 100 in a nanopore based sequencing chip. A membrane 102 is formed over the surface of the cell. In some embodiments, membrane 102 is a lipid bilayer. The bulk electrolyte 114 containing protein nanopore transmembrane molecular complexes (PNTMC) and the analyte of interest is placed directly onto the surface of the cell. A single PNTMC 104 is inserted into membrane 102 by electroporation. The individual membranes in the array are neither chemically nor electrically connected to each other. Thus, each cell in the array is an independent sequencing machine, producing data unique to the single polymer molecule associated with the PNTMC. PNTMC 104 operates on the analytes and modulates the ionic current through the otherwise impermeable bilayer.

With continued reference to Figure 1, analog measurement circuitry 112 is connected to a metal electrode 110 covered by a thin film of electrolyte 108. The thin film of electrolyte 108 is isolated from the bulk electrolyte 114 by the ion-impermeable membrane 102. PNTMC 104 crosses membrane 102 and provides the only path for ionic current to flow from the bulk liquid to working electrode 110. The cell also includes a counter electrode (CE) 116, which is an electrochemical potential sensor. The cell also includes a reference electrode 117.

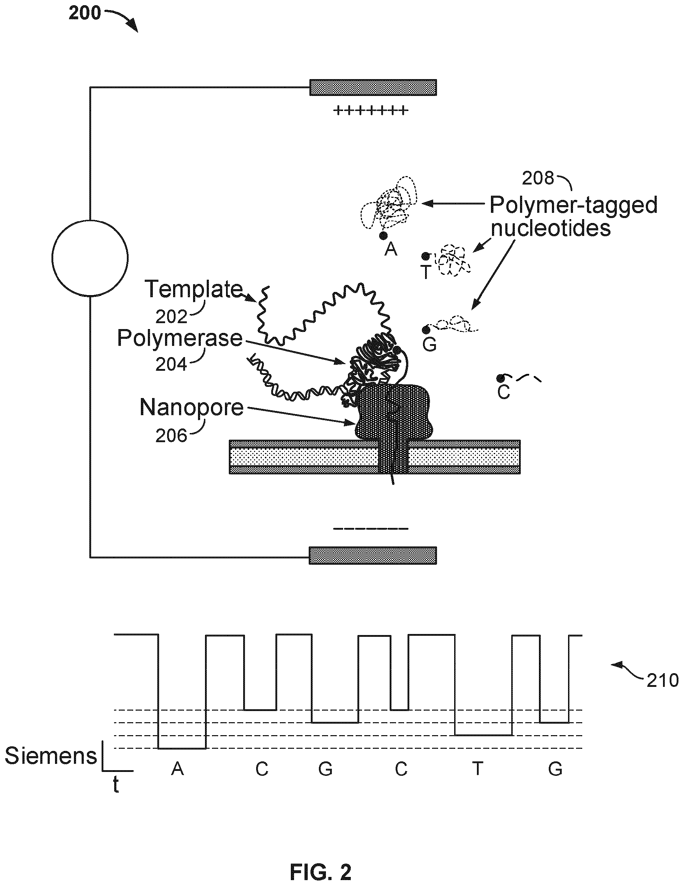

In some embodiments, a nanopore array enables parallel sequencing using the single molecule nanopore based sequencing by synthesis (Nano-SBS) technique. Figure 2 illustrates an embodiment of a cell 200 performing nucleotide sequencing with the Nano-SBS technique. In the Nano-SBS technique, a template 202 to be sequenced and a primer are introduced to cell 200. To this template-primer complex, four differently tagged nucleotides 208 are added to the bulk aqueous phase. As the correctly tagged nucleotide is complexed with the polymerase 204, the tail of the tag is positioned in the barrel of nanopore 206. The tag held in the barrel of nanopore 206 generates a unique ionic blockade signal 210, thereby electronically identifying the added base due to the tags' distinct chemical structures.

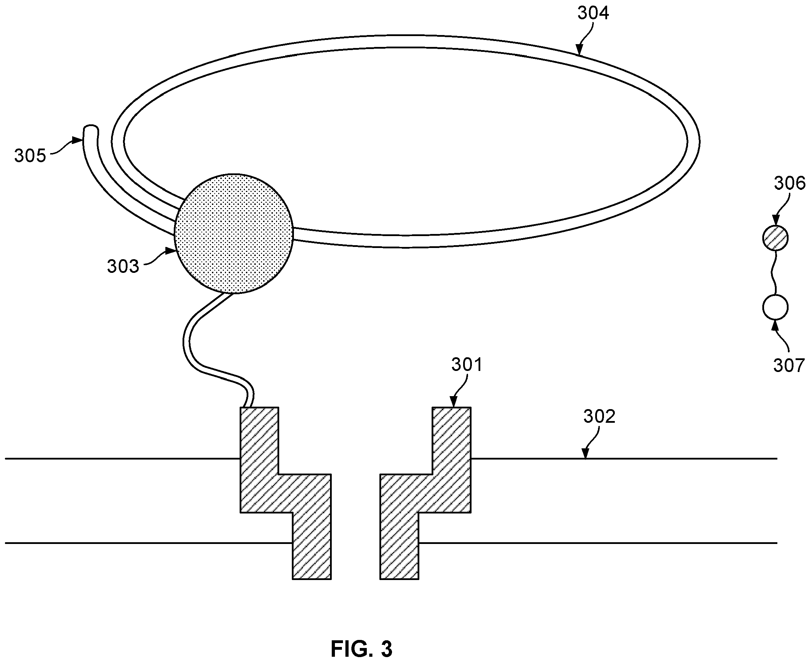

Figure 3 illustrates an embodiment of a cell about to perform nucleotide sequencing with pre-loaded tags. A nanopore 301 is formed in a membrane 302. An enzyme 303 (e.g., a polymerase, such as a DNA polymerase) is associated with the nanopore. In some cases, polymerase 303 is covalently attached to nanopore 301. Polymerase 303 is associated with a nucleic acid molecule 304 to be sequenced. In some embodiments, the nucleic acid molecule 304 is circular. In some cases, nucleic acid molecule 304 is linear. In some embodiments, a nucleic acid primer 305 is hybridized to a portion of nucleic acid molecule 304. Polymerase 303 catalyzes the incorporation of nucleotides 306 onto primer 305 using single stranded nucleic acid molecule 304 as a template. Nucleotides 306 comprise tag species ("tags") 307.

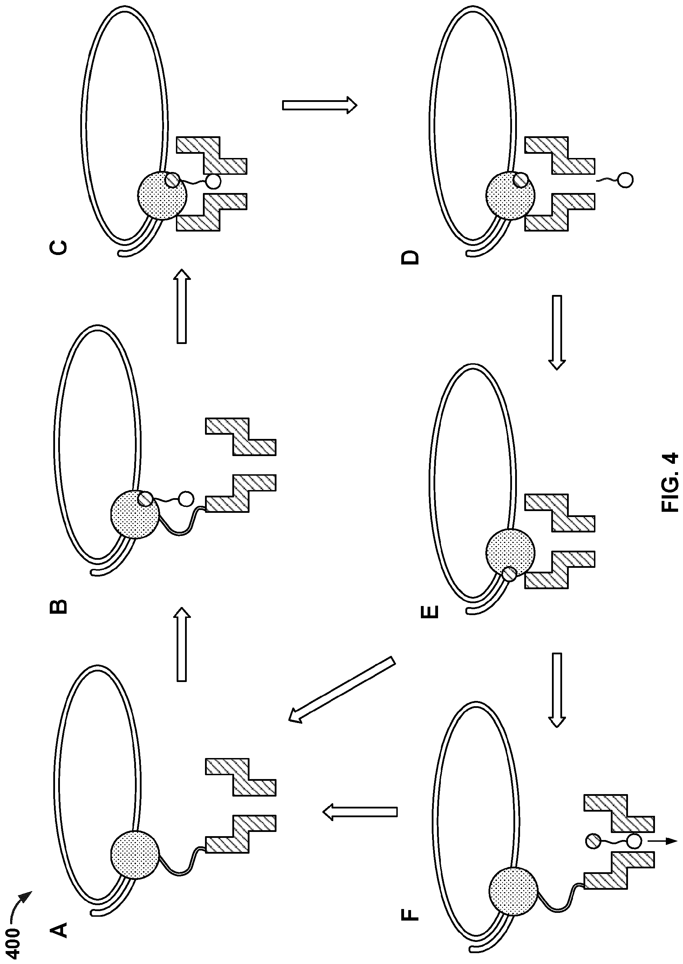

Figure 4 illustrates an embodiment of a process 400 for nucleic acid sequencing with pre-loaded tags. At stage A, a tagged nucleotide (one of four different types: A, T, G, or C) is not associated with the polymerase. At stage B, a tagged nucleotide is associated with the polymerase. At stage C, the polymerase is in close proximity to the nanopore. The tag is pulled into the nanopore by an electrical field generated by a voltage applied across the membrane and/or the nanopore.

Some of the associated tagged nucleotides are not base paired with the nucleic acid molecule. These non-paired nucleotides typically are rejected by the polymerase within a time scale that is shorter than the time scale for which correctly paired nucleotides remain associated with the polymerase. Since the non-paired nucleotides are only transiently associated with the polymerase, process 400 as shown in Figure 4 typically does not proceed beyond stage B.

Before the polymerase is docked to the nanopore, the conductance of the nanopore is ~300 pico Siemens (300 pS). At stage C, the conductance of the nanopore is about 60 pS, 80 pS, 100 pS, or 120 pS corresponding to one of the four types of tagged nucleotides. The polymerase undergoes an isomerization and a transphosphorylation reaction to incorporate the nucleotide into the growing nucleic acid molecule and release the tag molecule. In particular, as the tag is held in the nanopore, a unique conductance signal (e.g., see signal 210 in Figure 2) is generated due to the tag's distinct chemical structures, thereby identifying the added base electronically. Repeating the cycle (i.e., stage A through E or stage A through F) allows for the sequencing of the nucleic acid molecule. At stage D, the released tag passes through the nanopore.

In some cases, tagged nucleotides that are not incorporated into the growing nucleic acid molecule will also pass through the nanopore, as seen in stage F of Figure 4. The unincorporated nucleotide can be detected by the nanopore in some instances, but the method provides a means for distinguishing between an incorporated nucleotide and an unincorporated nucleotide based at least in part on the time for which the nucleotide is detected in the nanopore. Tags bound to unincorporated nucleotides pass through the nanopore quickly and are detected for a short period of time (e.g., less than 10 ms), while tags bound to incorporated nucleotides are loaded into the nanopore and detected for a long period of time (e.g., at least 10 ms).

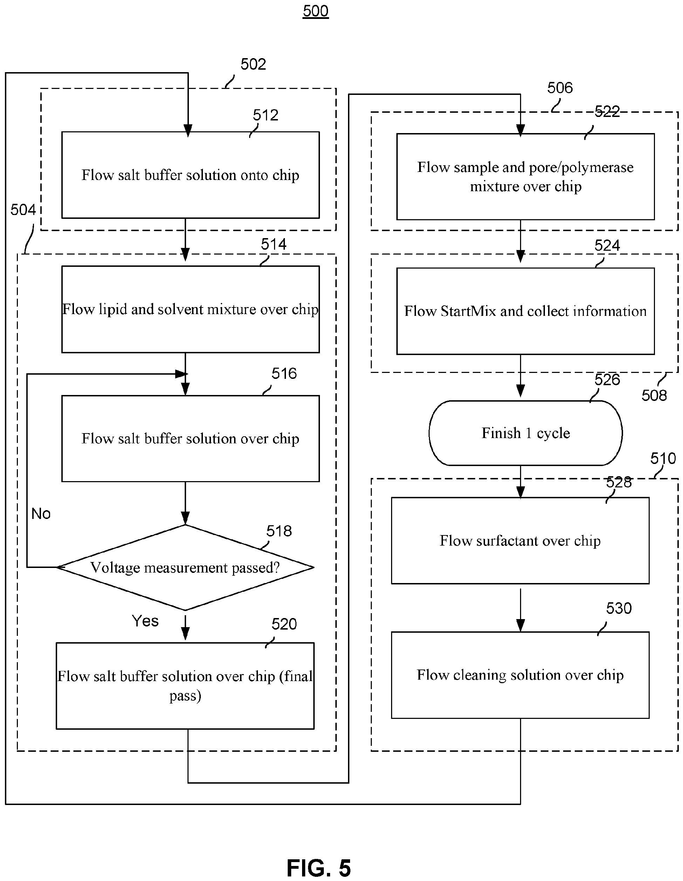

Figure 5 illustrates an embodiment of a fluidic workflow process 500 for flowing different types of fluids (liquids or gases) through the cells of a nanopore based sequencing chip during different phases of the chip's operation. The nanopore based sequencing chip operates in different phases, including an initialization and calibration phase (phase 502), a membrane formation phase (phase 504), a nanopore formation phase (phase 506), a sequencing phase (phase 508), and a cleaning and reset phase (phase 510).

At the initialization and calibration phase 502, a salt buffer is flowed through the cells of the nanopore based sequencing chip at 512. The salt buffer may be potassium choloride (KCl), potassium acetate (KAc), sodium trifluoroacetate (NaTFA), and the like.

At the membrane formation phase 504, a membrane, such as a lipid bilayer, is formed over each of the cells. At 514, a lipid and decane mixture is flowed over the cells. At 516, a salt buffer is flowed over the cells. At 518, voltage measurements across the lipid bilayers are made to determine whether the lipid bilayers are properly formed. If it is determined that the lipid bilayers are not properly formed, then step 516 is repeated; otherwise, the process proceeds to step 520. At 520, a salt buffer is again introduced.

At the nanopore formation phase 506, a nanopore is formed in the bilayer over each of the cells. At 522, a sample and a pore/polymerase mixture are flowed over the cells.

At the sequencing phase 508, DNA sequencing is performed. At 524, StartMix is flowed over the cells, and the sequencing information is collected and stored. StartMix is a reagent that initiates the sequencing process. After the sequencing phase, one cycle of the process is completed at 526.

At the cleaning and reset phase 510, the nanopore based sequencing chip is cleaned and reset such that the chip can be recycled for additional uses. At 528, a surfactant is flowed over the cells. At 530, ethanol is flowed over the cells. In this example, a surfactant and ethanol are used for cleaning the chip. However, alternative fluids may be used. Steps 528 and 530 may also be repeated a plurality of times to ensure that the chip is properly cleaned. After step 530, the lipid bilayers and pores have been removed and the fluidic workflow process 500 can be repeated at the initialization and calibration phase 502 again.

As shown in process 500 described above, multiple fluids with significantly different properties (e.g., compressibility, hydrophobicity, and viscosity) are flowed over an array of sensors on the surface of the nanopore based sequencing chip. For improved efficiency, each of the sensors in the array should be exposed to the fluids or gases in a consistent manner. For example, each of the different types of fluids should be flowed over the nanopore based sequencing chip such that the fluid or gas may be delivered to the chip, evenly coating and contacting each of the cells' surface, and then delivered out of the chip. As described above, a nanopore based sequencing chip incorporates a large number of sensor cells configured as an array. As the nanopore based sequencing chip is scaled to include more and more cells, achieving an even flow of the different types of fluids or gases across the cells of the chip becomes more challenging.

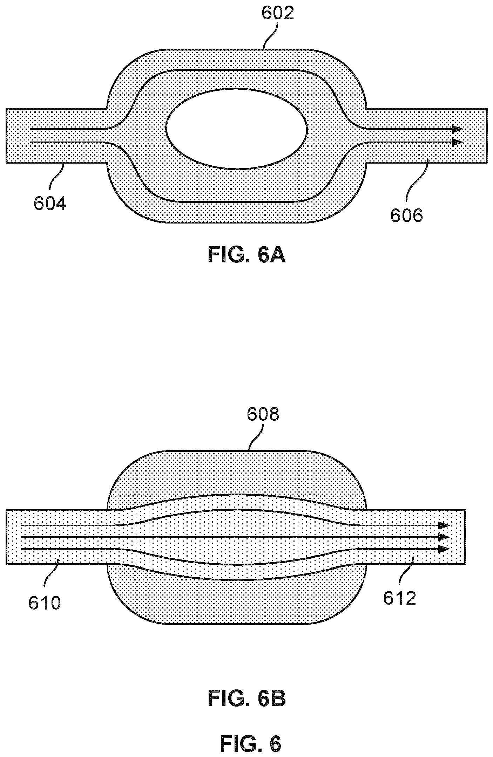

Figure 6A illustrates an exemplary flow of a fluid across the nanopore based sequencing chip. In Figure 6A, an inlet (e.g., a tube) 604 delivers a fluid to a nanopore based sequencing chip 602, and an outlet 606 delivers the fluid or gas out of the chip. Due to the difference in width between the inlet and the nanopore based sequencing chip, as the fluid or gas enters chip 602, the fluid or gas flows through paths that cover the cells that are close to the outer perimeter but not the cells in the center portion of the chip.

Figure 6B illustrates another exemplary flow of a fluid across the nanopore based sequencing chip. In Figure 6B, an inlet 610 delivers a fluid to a nanopore based sequencing chip 608, and an outlet 612 delivers the fluid or gas out of the chip. As the fluid or gas enters chip 608, the fluid or gas flows through paths that cover the cells that are close to the center portion of the chip but not the cells that are close to the outer perimeter of the chip.

As shown in Figure 6A and Figure 6B above, the nanopore based sequencing chip has one or more "dead" zones in the flow chamber. In the embodiment shown in Figure 6A, the dead zones are distributed close to the center of the chip. In the embodiment shown in Figure 6B, the dead zones are distributed close to the outer perimeter of the chip. The sensors in the chip array beneath the dead zones are exposed to a small amount of the fluid or a slow flow of the fluid, while the sensors outside of the dead zones are exposed to an excess or fast flow of the fluid.

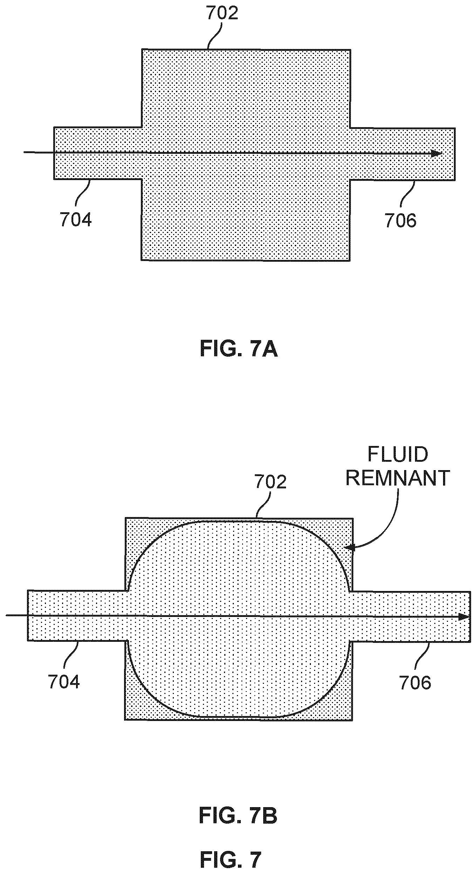

Furthermore, the introduction of a second fluid may not displace the first fluid in the dead zones effectively. Figure 7A illustrates an exemplary flow of a first type of fluid across the nanopore based sequencing chip. In this example, since the dead zones are located at the corners of the nanopore base sequencing chip, the corners of the chip are exposed to the first fluid later than other portions of the chip, but eventually the corners are finally filled up with the first fluid. Figure 7B illustrates that a second fluid is flowed through the chip after a first fluid has been flowed through the chip at an earlier time. Because the dead zones are located at the corners of the chip, the second fluid fails to displace the first fluid at the corners within a short period of time. As a result, the sensors in the array are not exposed to the right amount of fluid in a consistent manner.

The design of the flow chamber may also affect the formation of lipid bilayers with the appropriate thickness. With reference to step 514 of process 500 in Figure 5, a lipid and decane mixture is flowed over the cells, creating a thick lipid layer on top of each of the cells. In order to reduce the thickness of a lipid layer, in some embodiments, one or more air bubbles are flowed over the sensor to scrape the lipid layer into a thinner layer at step 516 of process 500. The design of the flow chamber should be optimized to control the scraping boundary between the air and the lipid layers, such that an even wiping action is performed over all of the sensors. In addition, the design of the flow chamber may be optimized to prevent the air bubbles from collapsing mid-way across the flow chamber; otherwise, only a portion of the lipid layers in the chip are scraped or "thinned."

With continued reference to Figure 6 and Figure 7, when the flow chamber flows the fluid from one end to the opposite end of the chip, the size of the dead zones within the chip and the collapsing of the air bubbles, in some embodiments, may be reduced by controlling the flow of the fluids and the air bubbles using different pressure and velocity. However, the improvement is limited.

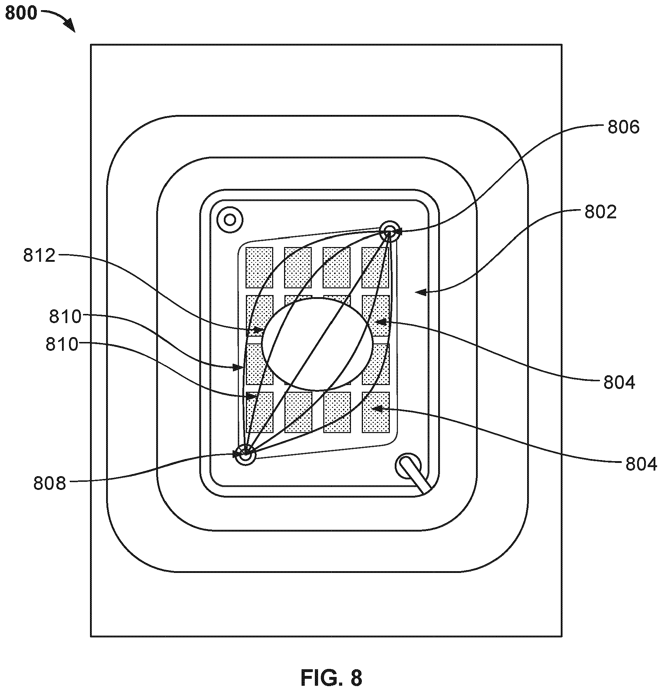

Figure 8 illustrates the top view of a nanopore based sequencing system 800 with a flow chamber enclosing a silicon chip that allows liquids and gases to pass over and contact sensors on the chip surface. In this example, the nanopore array chip 802 includes 16 sensor banks (804) in a 4 X 4 row-column arrangement. However, other arrangements of the sensors cells may be used as well. System 800 includes a counter electrode 812 positioned above the flow chamber. Fluids are directed from an inlet 806 to the flow chamber atop chip 802, and the fluids are directed out of the flow chamber via an outlet 808. The inlet and the outlet may be tubes or needles. Inlet 806 and outlet 808 are each positioned at one of two corners of the nanopore array chip 802 diagonally across from each other. Because the chamber is considerably wider than the inlet's width, as the fluid or gas enters the chamber, the fluid or gas flows through different paths 810 that cover more cells that are close to the center portion of the chip than cells that are close to the remaining two corners of the chip. The fluid or gas travels from one corner to another diagonal corner, leaving trapped fluids in dead zones in the remaining corners.

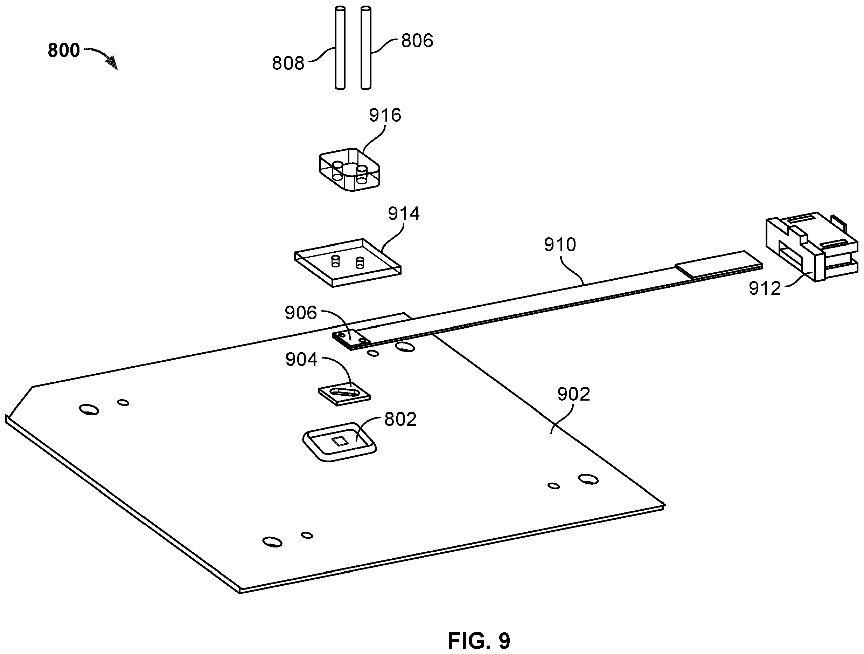

Figure 9 illustrates the various components that are assembled together to form the nanopore based sequencing system 800 as shown in Figure 8. System 800 includes various components, including a printed circuit board 902, a nanopore array chip 802, a gasket 904, counter and reference electrodes 906 connected by a flexible flat circuit 910 to a connector 912, a top cover 914, an inlet/outlet guide 916, an inlet 806, and an outlet 808.

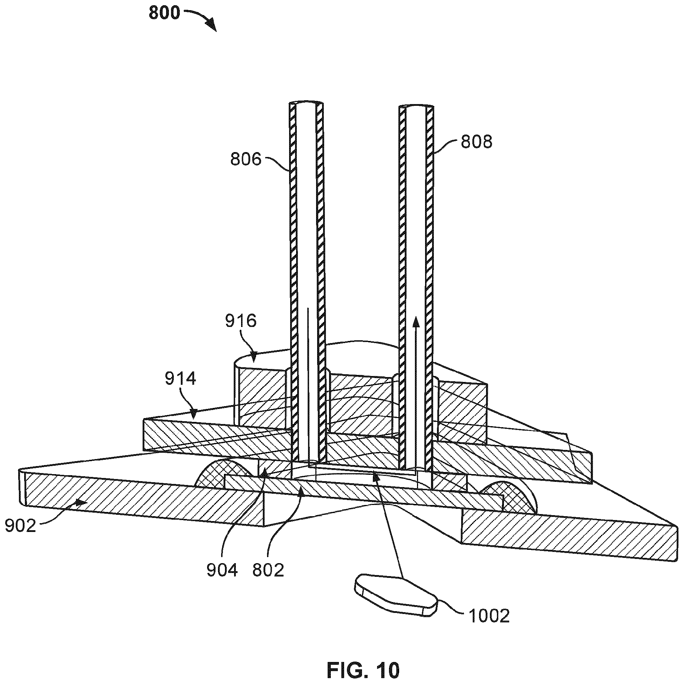

Figure 10 illustrates another exemplary view of nanopore based sequencing system 800. The flow chamber is the space formed between the top cover 914, the gasket 904, and the nanopore array chip 802. The chamber volume is shown as 1002 in Figure 10.

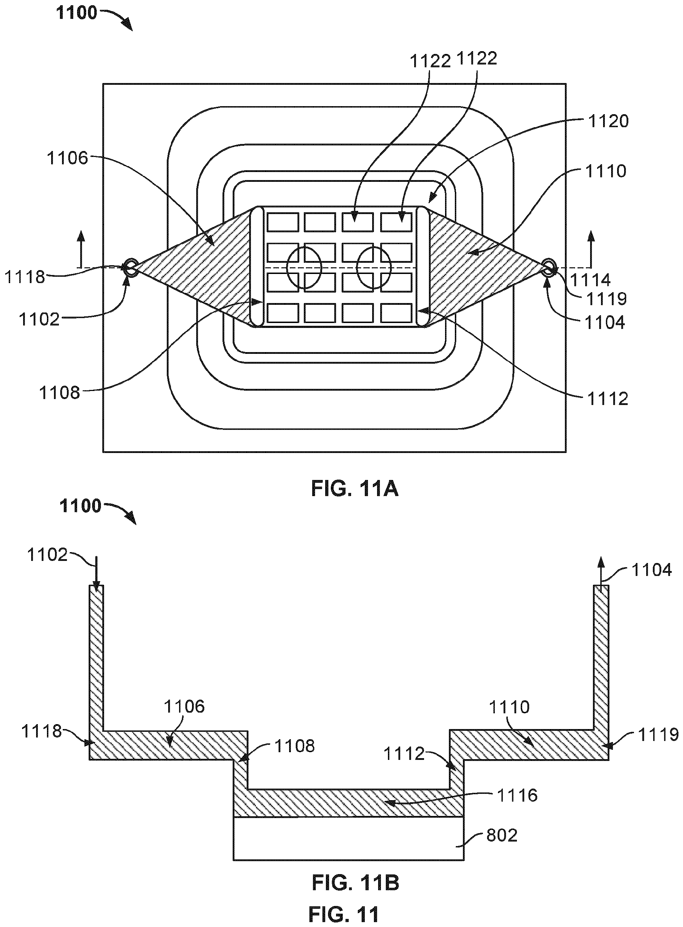

Figure 11A illustrates the top view of a nanopore based sequencing system 1100 with an improved flow chamber enclosing a silicon chip that allows liquids and gases to pass over and contact sensors on the chip surface. Figure 11B illustrates the cross sectional view of system 1100 from the position of a plane 1114 through the system.

A fluid is directed into system 1100 through an inlet 1102. Inlet 1102 may be a tube or a needle. For example, the tube or needle may have a diameter of one millimeter. Instead of feeding the fluid or gas directly into the flow chamber, inlet 1102 feeds the fluid or gas to a fan-out plenum space or reservoir 1106. As shown in the top view of system 1100 (Figure 11A), fan-out plenum 1106 directs the fluid or gas outwardly from a central point, a small orifice 1118 of inlet 1102 that intersects (see Figure 11B) with the fan-out plenum 1106. Fan-out plenum 1106 spreads out from orifice 1118 into a fanlike shape. For example, the fanlike shape as shown in Figure 11A is a substantially triangular shape. However, other similar shapes that direct the fluid or gas outwardly from the small orifice 1118 may be used as well. In one example, orifice 1118 is one millimeter wide, and fan-out plenum 1106 fans out to seven millimeters, the width of one row of four sensor banks 1122.

With reference to the cross sectional view of system 1100 (Figure 11B), the fluid or gas fills fan-out plenum 1106 first and then spills over and drains down a narrow slit or slot 1108 that intersects with a flow chamber 1116, like a waterfall. Flow chamber 1116 allows the fluid or gas to pass over and contact sensors on the surface of nanopore array chip 1120. Because slit 1108 spans across a row of sensor banks 1122, the fluid or gas is flowed more evenly across the sensor cells, reducing the number and areas of the dead zones within the chip. As the fluid or gas sweeps across the chip, the fluid or gas reaches a second narrow slit 1112 at the opposite end of the chip, and the fluid or gas is directed through slit 1112 up to a reverse fan-out plenum 1110. Reverse fan-out plenum 1110 directs the fluid or gas towards a central point, a small orifice 1119 of outlet 1104 that intersects (see Figure 11B) with the reverse fan-out plenum 1110. The fluid or gas is then directed out of system 1100 via an outlet 1104.

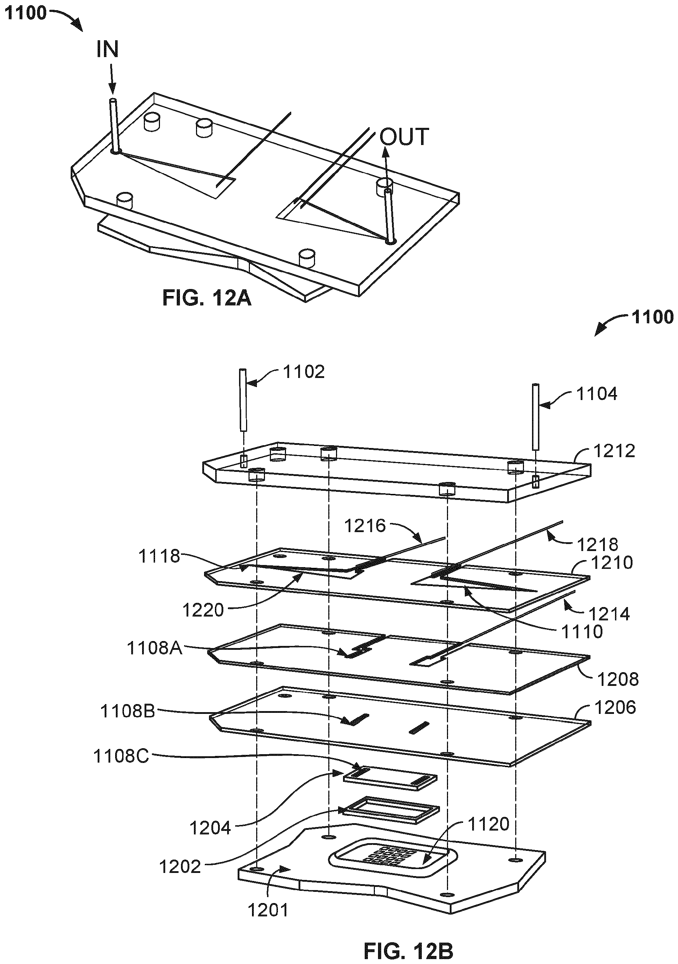

Figure 12A illustrates another exemplary view of nanopore based sequencing system 1100 with a fan-out plenum. Figure 12B illustrates the various components that are assembled together to form nanopore based sequencing system 1100 as shown in Figure 11. System 1100 includes various components, including a printed circuit board 1201, a nanopore array chip 1120, a gasket 1202, a gasket cover 1204, a middle plate 1206, a middle plate 1208, a reference electrode 1214, a middle plate 1210, a counter electrode 1218, a reference electrode 1216, a top plate 1212, an inlet 1102, and an outlet 1104.

The fan-out plenum is the space formed between top plate 1212, a fan-out void 1220 on the middle layer 1210, and middle layer 1208. Slit 1108 is the space formed by aligning a slit 1108A on middle plate 1208, a slit 1108B on middle plate 1206, and a slit 1108C on gasket cover 1204, and stacking middle plate 1208, middle plate 1206, and gasket cover 1204 on top of each other. The flow chamber is the space formed between gasket cover 1204, gasket 1202, and the nanopore array chip 1120.

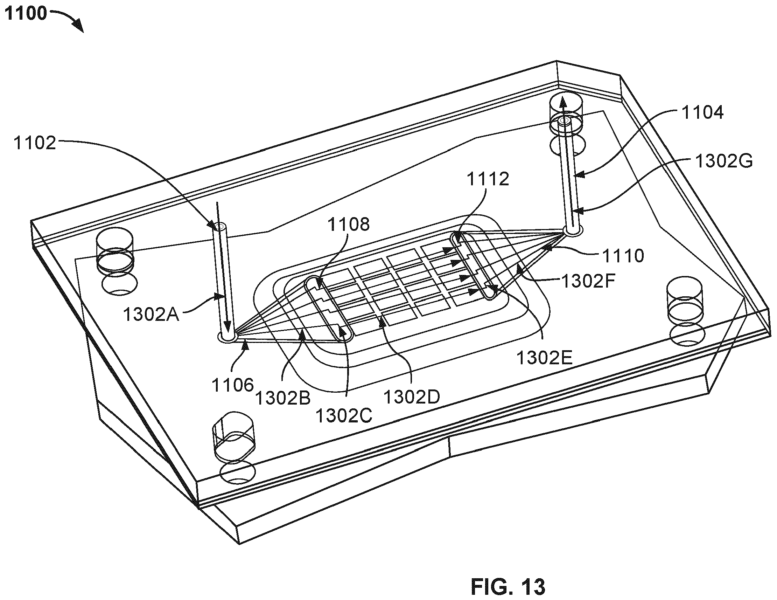

Figure 13 illustrates the paths that are followed by a fluid as it flows through the nanopore based sequencing system 1100 with a fan-out plenum. A fluid flows down inlet 1102 (see path 1302A), fills fan-out plenum 1106 first (see path 1302B) and then spills over and drains down slit 1108 (see path 1302C) that intersects with the flow chamber. The flow chamber allows the fluid or gas to pass over and contact sensors on the surface of the nanopore array chip as shown in path 1302D. Because slit 1108 spans across a row of sensor banks, the fluid or gas is flowed more evenly across the sensor cells, reducing the number and areas of the dead zones within the chip. As the fluid or gas sweeps across the chip, the fluid or gas reaches slit 1112 at the opposite end of the chip, and the fluid or gas is directed up through slit 1112 (see path 1302E) to a reverse fan-out plenum 1110. Reverse fan-out plenum 1110 converges the fluid or gas towards a central point (see path 1302F), a small orifice of outlet 1104 that intersects with reverse fan-out plenum 1110. The fluid or gas is then directed out of system 1100 via an outlet 1104 as shown in path 1302G.

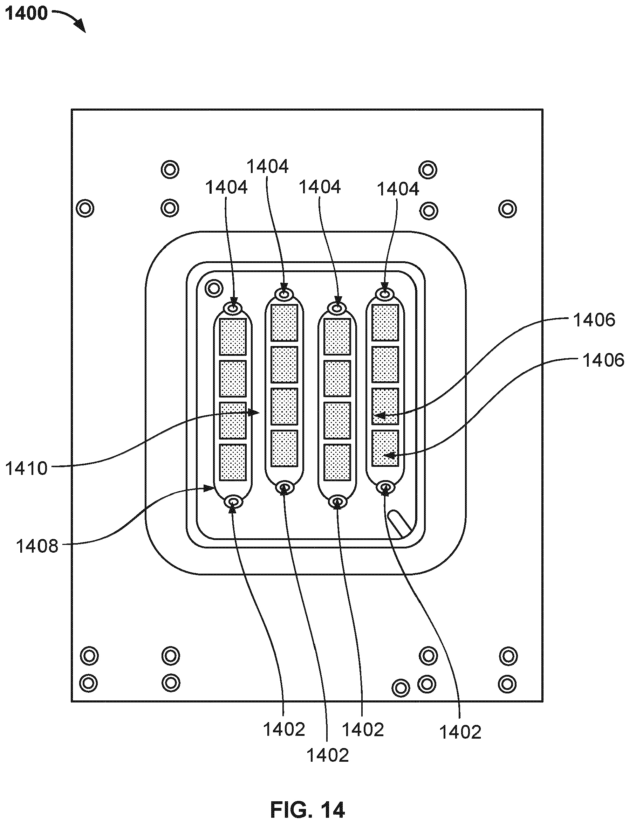

Figure 14 illustrates the top view of a nanopore based sequencing system 1400 with another improved flow chamber enclosing a silicon chip that allows liquids and gases to pass over and contact sensors on the chip surface. The flow chamber is divided into multiple channels 1408, each channel 1408 directing the fluids to flow directly above a single column (or a single row) of sensor banks 1406. As shown in Figure 14, system 1400 includes four inlets 1402 and four outlets 1404.

With reference to Figure 14, a fluid is directed into system 1400 in parallel through the four inlets 1402. Inlet 1402 may be a tube or a needle. For example, the tube or needle may have a diameter of one millimeter. Instead of feeding the fluid or gas directly into a wide flow chamber with a single continuous space, each of the inlets 1402 feeds the fluid or gas into a separate channel 1408 that directs the fluid or gas to flow directly above a single column of sensor banks 1406. The channels 1408 may be formed by stacking together a top plate and a gasket with dividers 1410 that divide the chamber into channels, and then mounting them on top of the chip. Once the fluid or gas flows through the channels 1408 to the opposite side of the chip, the fluid or gas is directed up in parallel through the four outlets 1404 and out of system 1400.

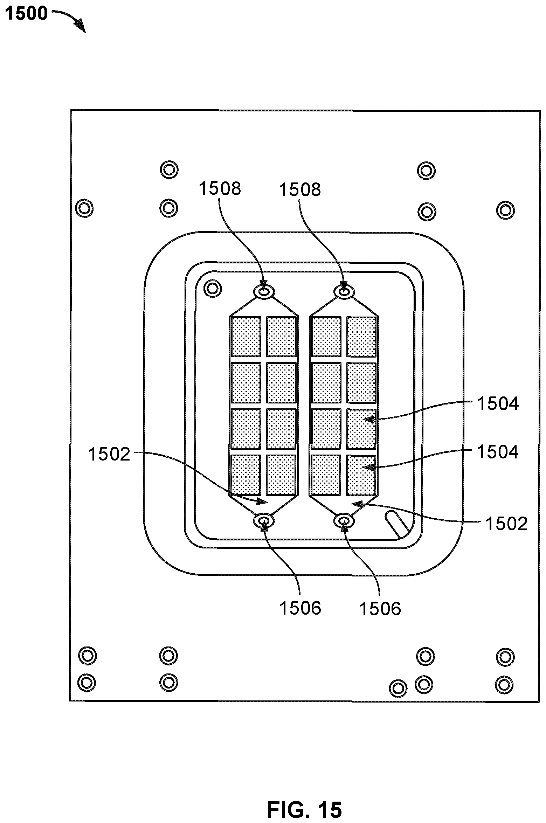

Figure 15 illustrates the top view of a nanopore based sequencing system 1500 with another improved flow chamber enclosing a silicon chip that allows liquids and gases to pass over and contact sensors on the chip surface. Similar to system 1400, the flow chamber in system 1500 is divided into multiple channels 1502, but each channel 1502 directs the fluids to flow directly above two columns (or two rows) of sensor banks 1504. The width of the channels is about 3.5 millimeters. As shown in Figure 15, system 1500 includes two inlets 1506 and two outlets 1508.

Both system 1400 and system 1500 allow the fluids to flow more evenly on top of all the sensors on the chip surface. The channel width is configured to be narrow enough such that capillary action has an effect. More particularly, the surface tension (which is caused by cohesion within the fluid) and adhesive forces between the fluid and the enclosing surfaces act to hold the fluid together, thereby preventing the fluid or the air bubbles from breaking up and creating dead zones. Therefore, when the width of a sensor bank is narrow enough, each of the flow channels may flow the fluids directly above two or more columns (or two or more rows) of sensor banks. In this case, system 1500 may be used. When the width of a sensor bank is not narrow enough, then each of the flow channels may flow the fluids directly above one column (or one row) of sensor banks only. In this case, system 1400 may be used.

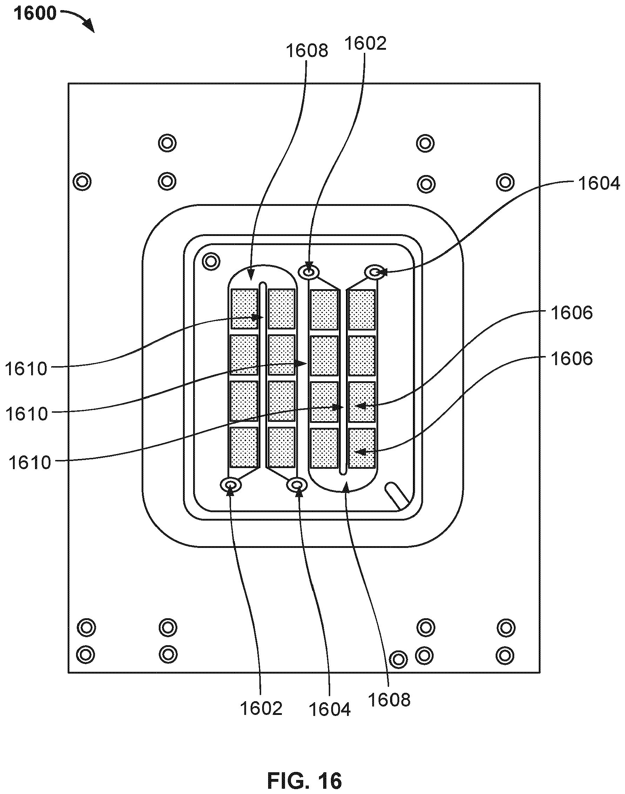

Figure 16 illustrates the top view of a nanopore based sequencing system 1600 with another improved flow chamber enclosing a silicon chip that allows liquids and gases to pass over and contact sensors on the chip surface. The flow chamber is divided into two horseshoe-shaped flow channels 1608, each channel 1608 directing the fluids to flow directly above a single column (or a single row) of sensor banks 1606 from one end of the chip to the opposite end and then directing the fluids to loop back and flow directly above a second adjacent column of sensor banks to the original end of the chip. As shown in Figure 16, system 1600 includes two inlets 1602 and two outlets 1604.

With reference to Figure 16, a fluid is directed into system 1600 in parallel through the two inlets 1602. Inlet 1602 may be a tube or a needle. For example, the tube or needle may have a diameter of one millimeter. Instead of feeding the fluid or gas directly into a wide flow chamber with a single continuous space, each of the inlets 1602 feeds the fluid or gas into a separate channel 1608 that directs the fluid or gas to flow directly above a single column of sensor banks 1606. The channels 1608 may be formed by stacking together a top plate and a gasket with dividers 1610 that divide the chamber into channels, and then mounting them on top of the chip. Once the fluid or gas flows through the channels 1608, the fluid or gas is directed up in parallel through the two outlets 1604 and out of system 1600.

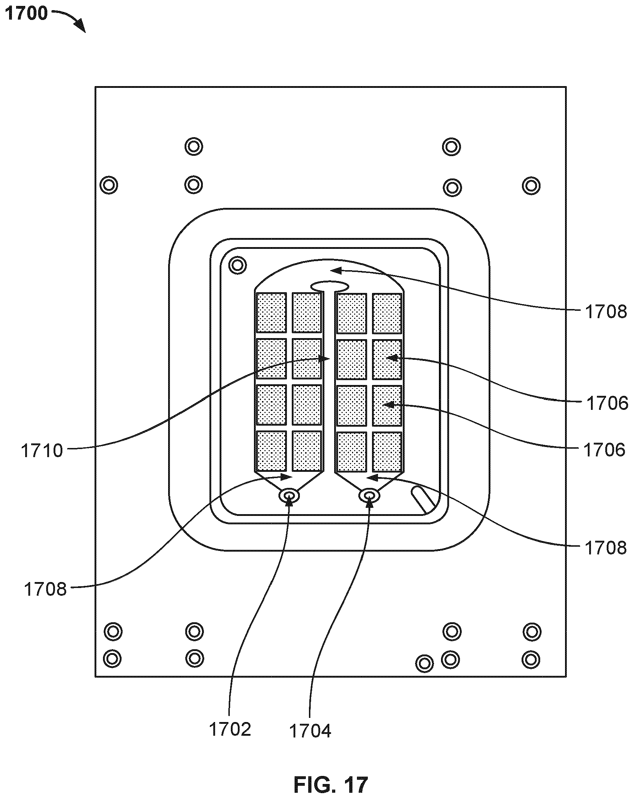

Figure 17 illustrates the top view of a nanopore based sequencing system 1700 with another improved flow chamber enclosing a silicon chip that allows liquids and gases to pass over and contact sensors on the chip surface. Similar to system 1600, the flow chamber in system 1700 includes a horseshoe-shaped flow channel 1708, but horseshoe-shaped flow channel 1708 directs the fluids to flow directly above two columns (or two rows) of sensor banks 1706. The width of the channel is about 3.5 millimeters. As shown in Figure 17, system 1700 includes an inlet 1702 and an outlet 1704.

Both system 1700 and system 1700 allow the fluids to flow more evenly on top of all the sensors on the chip surface. The channel width is configured to be narrow enough such that capillary action has an effect. More particularly, the surface tension (which is caused by cohesion within the fluid) and adhesive forces between the fluid and the enclosing surfaces act to hold the fluid together, thereby preventing the fluid or the air bubbles from breaking up and creating dead zones. Therefore, when the width of a sensor bank is narrow enough, each of the horseshoe-shaped flow channels may flow the fluids directly above two or more columns (or two or more rows) of sensor banks. In this case, system 1700 may be used. When the width of a sensor bank is not narrow enough, then each of horseshoe-shaped flow channels may flow the fluids directly above one column (or one row) of sensor banks only. In this case, system 1600 may be used.

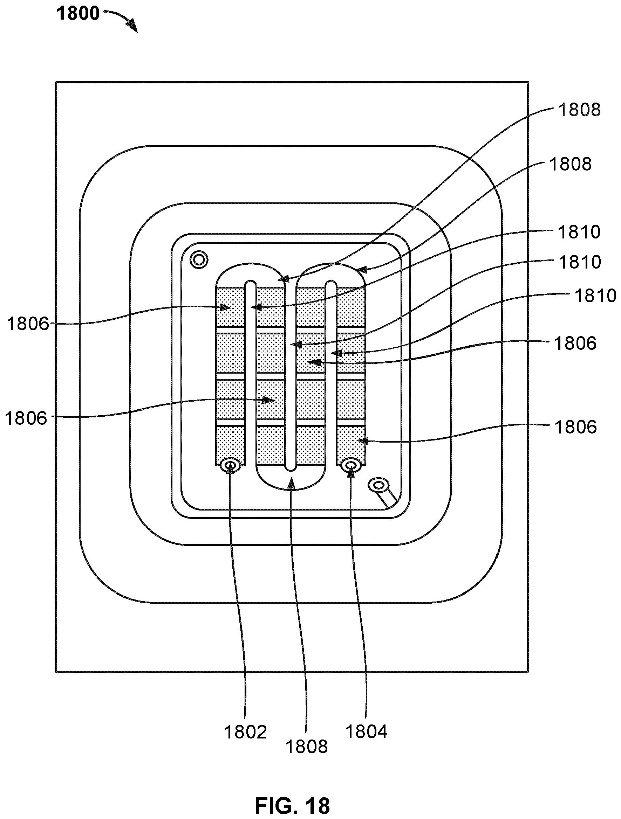

In some embodiments, the nanopore based sequencing system includes an improved flow chamber having a serpentine fluid flow channel that directs the fluids to traverse over different sensors of the chip along the length of the channel. Figure 18 illustrates the top view of a nanopore based sequencing system 1800 with an improved flow chamber enclosing a silicon chip that allows liquids and gases to pass over and contact sensors on the chip surface. The flow chamber includes a serpentine or winding flow channel 1808 that directs the fluids to flow directly above a single column (or a single row) of sensor banks 1806 from one end of the chip to the opposite end and then directs the fluids to repeatedly loop back and flow directly above other adjacent columns of sensor banks until all of the sensor banks have been traversed at least once. As shown in Figure 18, system 1800 includes an inlet 1802 and an outlet 1804 and the serpentine or winding flow channel 1808 directs a fluid to flow from the inlet 1802 to the outlet 1804 over all 16 sensor banks 1806.

With reference to Figure 18, a fluid is directed into system 1800 through inlet 1802. Inlet 1802 may be a tube or a needle. For example, the tube or needle may have a diameter of one millimeter. Instead of feeding the fluid or gas directly into a wide flow chamber with a single continuous space, inlet 1802 feeds the fluid or gas into a serpentine flow channel 1808 that directs the fluid or gas to flow directly above the columns of sensor banks 1806 serially connected together through serpentine flow channel 1808. The serpentine channel 1808 may be formed by stacking together a top plate and a gasket with dividers 1810 that divide the chamber into the serpentine channel, and then mounting them on top of the chip. Once the fluid or gas flows through the serpentine channel 1808, the fluid or gas is directed up through outlet 1804 and out of system 1800.

System 1800 allows the fluids to flow more evenly on top of all the sensors on the chip surface. The channel width is configured to be narrow enough such that capillary action has an effect. More particularly, the surface tension (which is caused by cohesion within the fluid) and adhesive forces between the fluid and the enclosing surfaces act to hold the fluid together, thereby preventing the fluid or the air bubbles from breaking up and creating dead zones. For example, the channel may have a width of 1 millimeter or less. The narrow channel enables controlled flow of the fluids and minimizes the amount of remnants from a previous flow of fluids or gases.

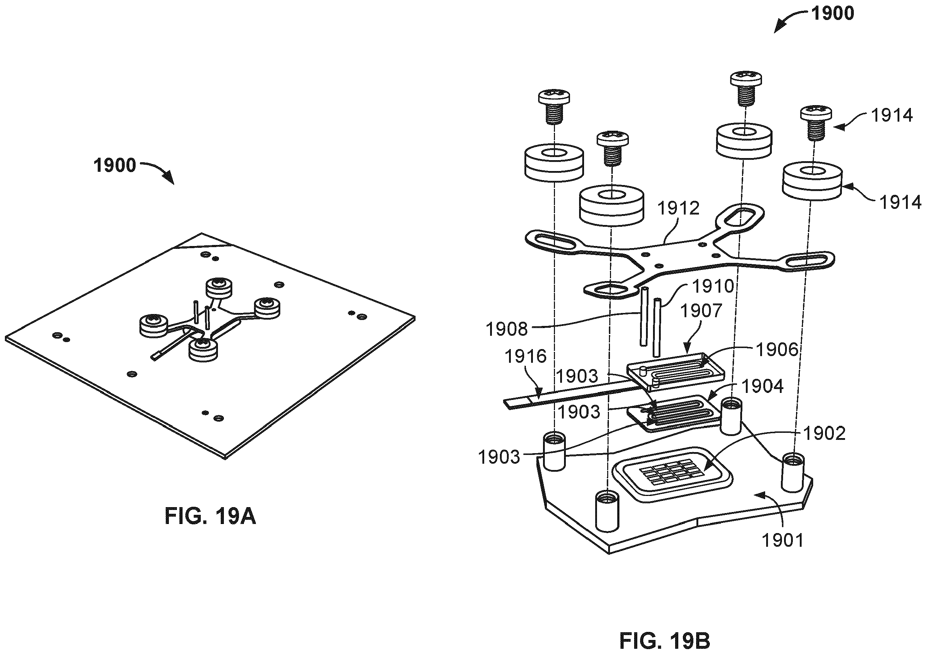

Figure 19A illustrates an exemplary view of one embodiment of a nanopore based sequencing system 1900 with a serpentine flow channel. Figure 19B illustrates the various components that are laminated together to form nanopore based sequencing system 1900. System 1900 includes various components, including a printed circuit board 1901, a nanopore array chip 1902, a gasket 1904 with dividers 1903, a backing plate 1907, a counter electrode 1906 on the underside of backing plate 1907, a flexible flat circuit 1916 connecting to counter electrode 1906, an inlet 1908, an outlet 1910, a spring plate 1912, and a plurality of fastening hardware 1914. The serpentine flow channel is the space formed between backing plate 1907, gasket 1904, and nanopore array chip 1902.

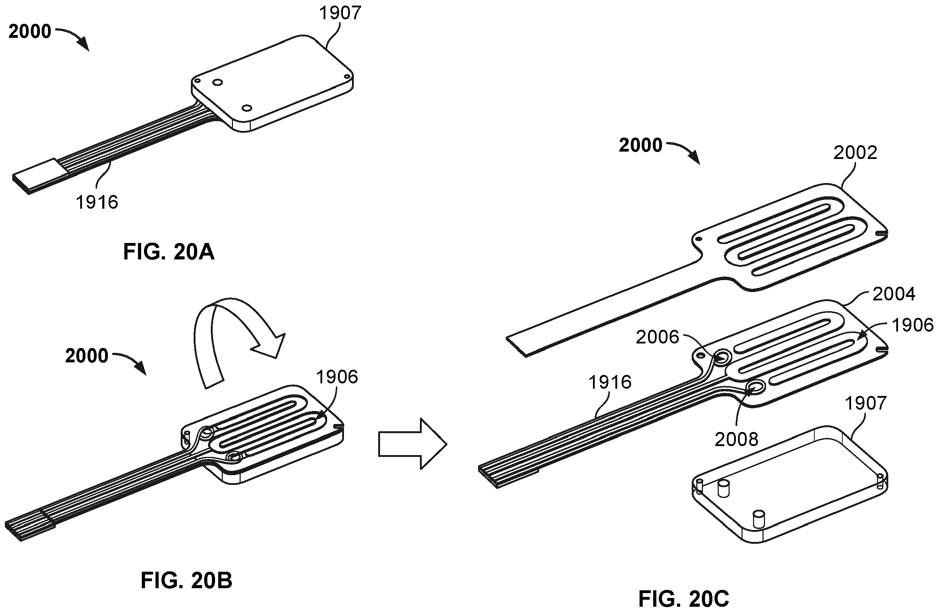

Figure 20A illustrates the top side view of a backing plate and a flexible flat circuit that is connected to the counter electrode (not visible) located on the bottom side of the backing plate. Figure 20B illustrates the same unit 2000 as shown in Figure 20A when the backing plate is flipped upside down. As shown in this figure, the counter or common electrode 1906 has a serpentine, spiral, or winding shape. Referring back to Figure 19B, the counter electrode's serpentine shape matches with the serpentine channel of gasket 1904, such that the counter electrode is positioned directly above the sensor banks without being blocked by the dividers 1903 of the gasket. The dividers 1903 are disposed between the sensor banks so that the dividers do not block the flow of the fluids or gases over the sensor banks.

Figure 20C illustrates the various components of unit 2000 that are laminated together. Unit 2000 includes a dielectric layer 2002, a counter electrode 1906 on a film 2004, a reference electrode 2006, a reference electrode 2008, a flexible flat circuit 1916, and a backing plate 1907.

Figure 19B and Figure 20C illustrate that the flow channel is formed by laminating a backing plate with the counter electrode, a gasket, and the silicon chip together. However, the backing plate with the counter electrode and the gasket may be integrated together as a single unit made of the electrode material, and the unit is machined to form the serpentine flow channel.

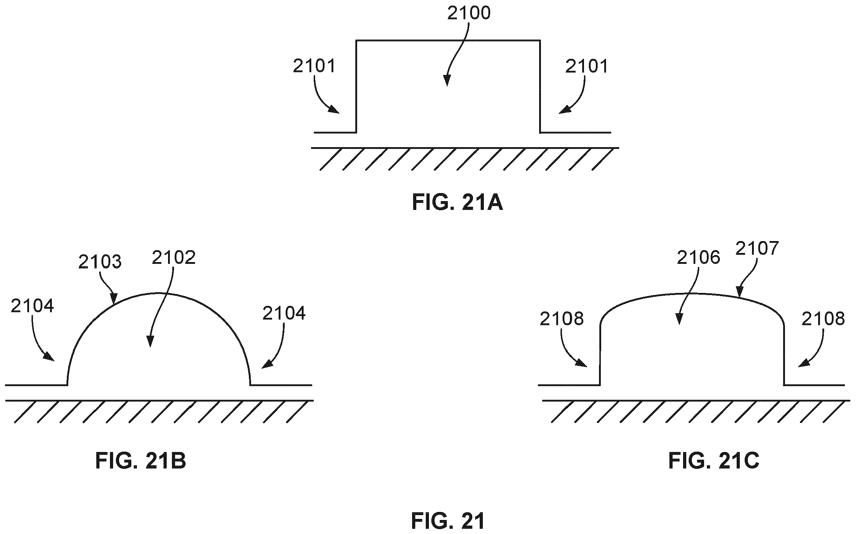

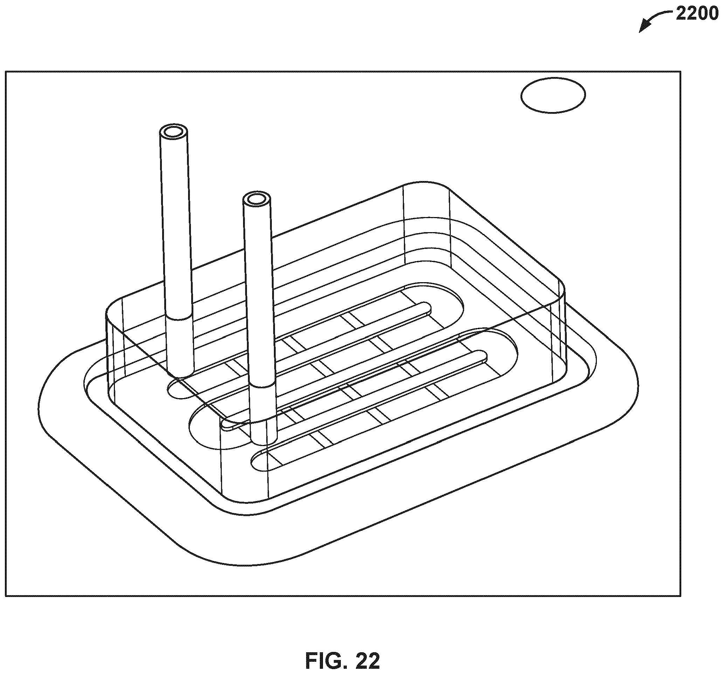

Besides the geometry and dimensions of the flow chamber, other features may also facilitate a more even flow of the fluids on top of all the sensors on the chip surface. Figure 21A illustrates a cross sectional view of a flow channel 2100 with sharp edges or sharp corners that may trap fluids more easily. Figure 21B illustrates a cross sectional view of a flow channel 2102 that has a curved roof 2103 and a D-shaped cross-sectional geometry. The sharp edges or sharp corners are replaced by round and smooth surfaces. Figure 21C illustrates a cross sectional view of another flow channel 2106 that has a curved roof 2107. Figure 22 illustrates a side view of a nanopore based sequencing system 2200 with flow channels having a D-shaped cross sectional geometry.

Another factor that affects the flow of the fluids on top of all the sensors on the chip surface is the height of the flow channel. For example, the height of the flow channel should be limited to one millimeter or below. In one embodiment, the height of the flow channel is 0.25 millimeters. Other factors that affect the flow of the fluids on top of all the sensors on the chip surface include the surface characteristics of the surfaces defining the flow channel, the flow rate of the fluids, the pressure of the fluid and the gases, and the like.

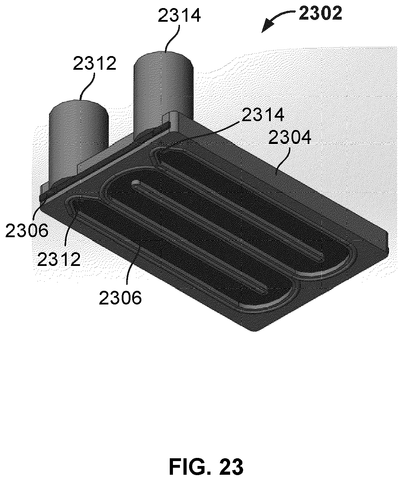

Figure 23 is a diagram illustrating an embodiment of a molded flow channel component. In some embodiments, the component shown in Figure 23 forms at least a portion of a flow channel shown in Figure 18. As shown in Figure 19B, gasket 1904 with dividers 1903 must be carefully aligned with counter electrode 1906 on the underside of backing plate 1907 during assembly to correctly assemble the flow channel. Additionally, as shown in Figure 20C, dielectric layer 2002, counter electrode 1906, film 2004, and backing plate 1907 must be carefully aligned during assembly. Requiring such a large number of components to be aligned together during assembly introduces complexities that may increase manufacturing cost and risk of error. Therefore, there exists a need for an efficient and reliable way to form the flow channel/chamber of a nanopore system.

Molded flow channel component 2302 includes molded portion 2304 and counter electrode portion 2306. When molded flow channel component 2302 is placed on a nanopore array chip (e.g., chip 1902) and secured, the serpentine void shown in molded flow channel component 2302 (e.g., counter electrode portion 2306 is exposed through the serpentine void of the molded portion 2304) becomes a flow chamber of a fluid flow channel that directs fluids to traverse over different sensors of the nanopore array chip. Molded portion 2304 includes shown raised dividers that guide the fluid flow along the length of the serpentine channel. Molded flow channel component 2302 includes inlet 2312 and outlet 2314. Both inlet 2312 and outlet 2314 include a tubular channel that provides fluid/gas flow in or out of the fluid flow chamber/channel. The inlet/outlet tubular channels are formed at least in part by molded portion 2304 and pass through counter electrode portion 2306.

Molded portion 2304 has been molded over counter electrode portion 2306. For example, counter electrode portion 2306 was placed inside a mold and molding material is injected into the mold to form molded portion 2304 over counter electrode portion 2306 to create molded flow channel component 2302. An example material of molded portion 2304 is an elastomer. An example of counter electrode portion 2306 is a metal (e.g., titanium nitride sputtered/coated stainless steel).

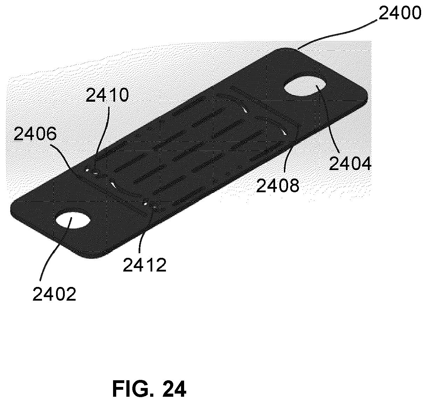

Figure 24 is a diagram illustrating an embodiment of a counter electrode insert. In some embodiments, counter electrode insert 2400 becomes counter electrode portion 2306 of Figure 23 after assembly. Counter electrode insert 2400 may be made of metal (e.g., stainless steel, steel, aluminum, etc.), semiconductor material (e.g., doped silicon) or other conductive material that may be rigid or flexible (e.g., foil). In some embodiments, counter electrode insert 2400 has been coated and/or sputtered with an electrically conductive material. For example, titanium nitride (TiN) has been sputtered on to a base material (e.g., glass, stainless steel, metal, silicon, etc.) to coat counter electrode insert 2400. This allows the portion of the counter electrode insert 2400 that is exposed inside a flow channel of molded flow channel component 2302 (e.g., portion exposed to chemical reagents) to be titanium nitride. In some embodiments, TiN is a preferred material because of its certain beneficial electrochemical properties, its general availability in and compatibility with existing standard semiconductor manufacturing processes, its compatibility with the biological and chemical reagents, and its relative low cost.

Counter electrode insert 2400 includes cutouts 2402 and 2404 that allow counter electrode insert 2400 to be aligned and stabilized inside an injection mold. Cutouts 2402 and 2404 are on tab ends of counter electrode insert 2400 that are removed (e.g., snapped off) after molding and discarded from counter electrode insert 2400 to form counter electrode portion 2306 of Figure 23. Cutouts 2406 and 2408 allow the tab ends to be more easily removed (e.g., snapped off) in the direction of the length of the cutouts 2406 and 2408. The tab ends allow easier alignment and stabilization inside a mold as well as easier handling of the component during molding. Cutout 2410 corresponds to inlet 2312 of Figure 23 and cutout 2412 corresponds to outlet 2314 of Figure 23 that allows a fluid/gas to pass through counter electrode insert 2400 via tubular channels and in/out of a fluid flow chamber/channel. The other cutouts shown in counter electrode insert 2400 couple counter electrode insert 2400 with a molding material by allowing the molding material to flow through and remain molded in the cutouts to secure the coupling between the counter electrode insert 2400 and the molding material. These other cutouts are placed in locations on the counter electrode insert 2400 to avoid surface portions that are to be exposed inside the fluid chamber/channel. A laser cutter may be utilized as well to produce the shown cutouts. In various embodiments, insert 2400 may have been stamped, chemically etched or cut using a water-jet to produce the shown cutouts.

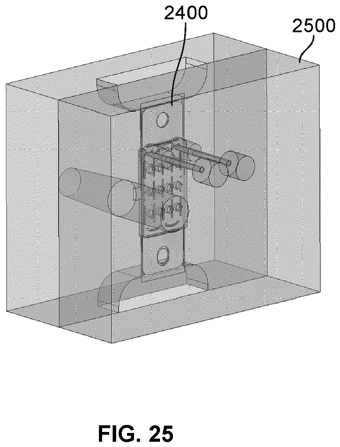

Figure 25 is a diagram illustrating an embodiment of a mold for a molded flow channel component. In some embodiments, mold 2500 is utilized to produce molded flow channel component 2302 of Figure 23. A transparent view of mold 2500 is shown to illustrate the internal structure of mold 2500. Mold 2500 includes a plurality of sectional pieces that are joined together when molding a component. These pieces may be separated after molding to extract the molded component. Counter electrode insert 2400 has been placed inside mold 2500. Mold 2500 includes tube features that are inserted in cutouts 2410 and 2412 of counter electrode insert 2400 to produce inlet/outlet tubular channels of molded flow channel component 2302. Counter electrode insert 2400 is secured inside mold 2500 via its tab ends, one or more cutouts and features of mold 2500 contacting a surface of counter electrode insert 2400. A molding material is injected into mold 2500 to injection mold the molding material around counter electrode insert 2400 and in the shape of mold 2500. In various embodiments, molding material may be an elastomer, rubber, silicone, polymer, thermoplastics, plastics, or any other injection moldable material. One or more cutouts/holes on counter electrode insert 2400 may be filled with the molding material to couple the molding material with counter electrode insert 2400. In some embodiments, the molding material injected into mold 2500 becomes molded portion 2304 of Figure 23. In various other embodiments, other molding techniques such as liquid injection molding (LIM), transfer molding, or compression molding are utilized to produce molded flow channel component 2302 of Figure 23.

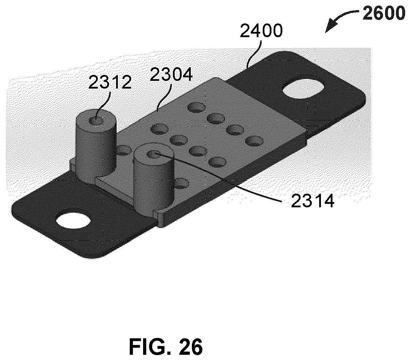

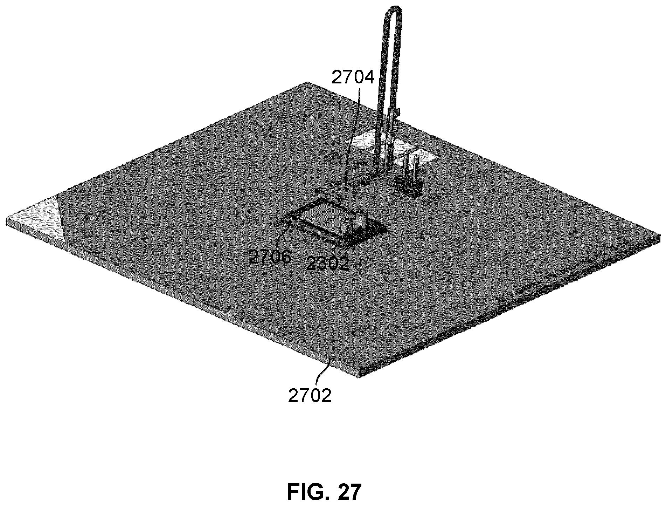

Figure 26 is a diagram illustrating an embodiment of a molded flow channel component removed from a mold. Component 2600 has been removed from mold 2500 after injection molding. After removing (e.g., bending/snapping off) shown end tabs of counter electrode insert 2400, component 2600 becomes molded flow channel component 2302 of Figure 23. The four by three array of openings/holes on top of molded portion 2304 exposes surfaces of counter electrode insert 2400. In some embodiments, this array of openings/holes is an artifact resulting from the twelve features/pins of mold 2500 that are used to hold and stabilize counter electrode insert 2400 in place in the mold during molding. In some embodiments, at least some of these openings/holes of the array are utilized to make electrical contact with a counter electrode portion. For example, a spring contact is placed in some of the openings/holes of the array and connected to an electrical potential source of a circuit board (e.g., shown in Figure 27).

Figure 27 is a diagram illustrating a portion of an embodiment of a nanopore based sequencing system utilizing a molded flow channel component. Molded flow channel component 2302 has been placed on top of a nanopore array chip that is mounted on circuit board 2702. Spring contact wire component 2704 is to be placed in the array of openings/holes on top of molded flow channel component 2302 to make electrical and physical contact with its counter electrode portion. The other end of spring contact wire component 2704 is to be connected to circuit board 2702 to provide an electrical potential source for its counter electrode. In some embodiments, the molded flow channel component 2302 and electrical contact of spring contact wire component 2704 of the counter electrode are secured and clamped via a clamp plate (e.g., without use of adhesives) that clamps spring contact wire component 2704 to molded flow channel component 2302. In some embodiments, the clamp plate also clamps molded flow channel component 2302 to the nanopore array chip to provide the compression required to seal and couple them together in creating a flow channel/chamber between molded flow channel component 2302 and the nanopore array chip. Encapsulate berm 2706 encapsulates wire bonds between electrical terminals of the nanopore array chip and circuit board 2702.

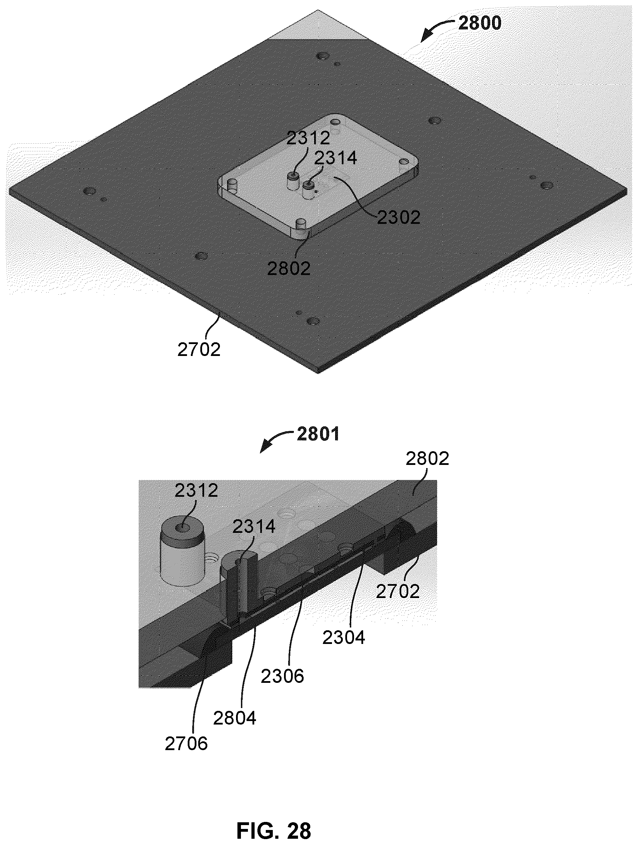

Figure 28 is a diagram illustrating an embodiment of a clamping of nanopore based sequencing system components. The views shown in Figure 28 have been simplified to illustrate the embodiment clearly. View 2800 shows an overview of the components of Figure 27 that have been clamped together.

Clamping plate 2802 clamps together molded flow channel component 2302 over a nanopore array chip. Clamping plate 2802 may be fastened and clamped to circuit board 2702 via screws or other coupling mechanisms. Clamping plate 2802 includes holes to accommodate and allow inlet 2312 and outlet 2314 to pass through clamping plate 2802. Clamping plate 2802 clamps the molded flow channel component to the nanopore array chip to provide the compression required to seal and couple them together in creating a flow channel/chamber without the use of adhesives and/or permanent/physical bonding to couple together the molded flow channel component to the nanopore array chip. Spring contact wire component 2704 is not shown to simplify the diagram but exists in the embodiment under clamping plate 2802. Clamping plate 2802 also secures the spring contact wire component to the counter electrode of the molded flow channel component via compression.

View 2801 shows a cutaway view of the embodiment shown in view 2800. Clamping plate 2802 is secured to circuit board 2702 and clamps together nanopore array chip 2804 with counter electrode portion 2306 and molded portion 2304 of molded flow channel component 2302. The gap between counter electrode portion 2306 and nanopore array chip 2804 is a part of a flow chamber/channel that holds and directs the fluids to traverse over different sensors of nanopore array chip 2804. The clamping force/pressure seals contact between molded portion 2304 and nanopore array chip 2804. In some embodiments, the material of molded portion 2304 is compliant to provide the seal under clamping force/pressure.

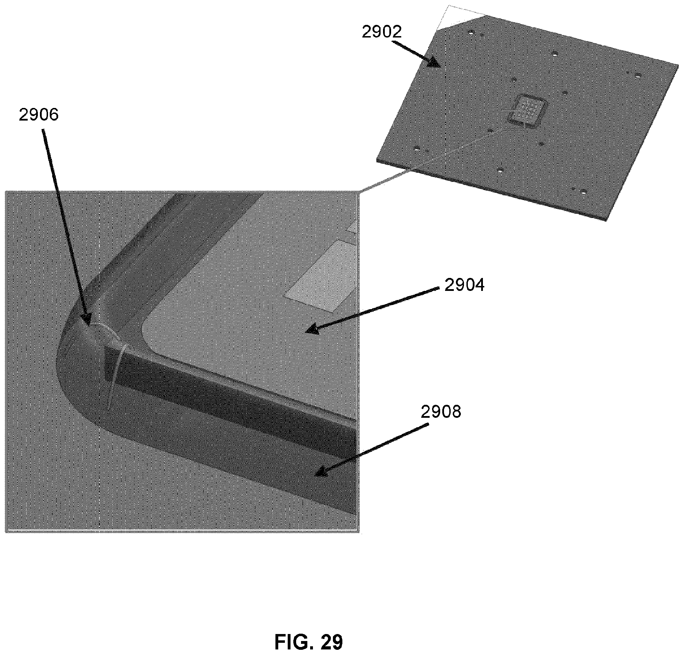

Figure 29 is a diagram illustrating an embodiment of encapsulated wire bonds. Wire bonds 2906 electrically connect nanopore based sequencing chip 2904 to circuit board 2902. Wire bonds 2906 are protected by encapsulant 2908. Although only two wire bonds have been shown to simplify the diagram, other wire bonds 2906 all around the perimeter of chip 2904 may be utilized to electrically connect chip 2904 to circuit board 2902. In order to protect wire bonds 2906 from damage and wetting, encapsulant 2908 (e.g., adhesive) is utilized to encapsulate wire bonds 2906 by applying a ring of encapsulant around the perimeter of chip 2904. In some embodiments, encapsulant 2908 is the encapsulant shown as surrounding a nanopore chip in other figures. However, the applied encapsulant cannot encroach on portions of the chip where one or more flow channel components are to be coupled to the chip.

Thus care must be taken to minimize the encroachment of the encapsulant onto the surface of the chip in order to maximize the area available on the chip surface for coupling the one or more flow channel components onto the chip. In various embodiments, the one or more flow channel components may be coupled to the chip surface by room temperature bonding, adhesive bonding, and/or compression bonding. During manufacturing, two distinct steps may need to be performed - first, encapsulating the wire bonds with careful attention paid to preventing undesired encroachment of the encapsulant onto the chip and then coupling and sealing the one or more flow channel components onto the chip.

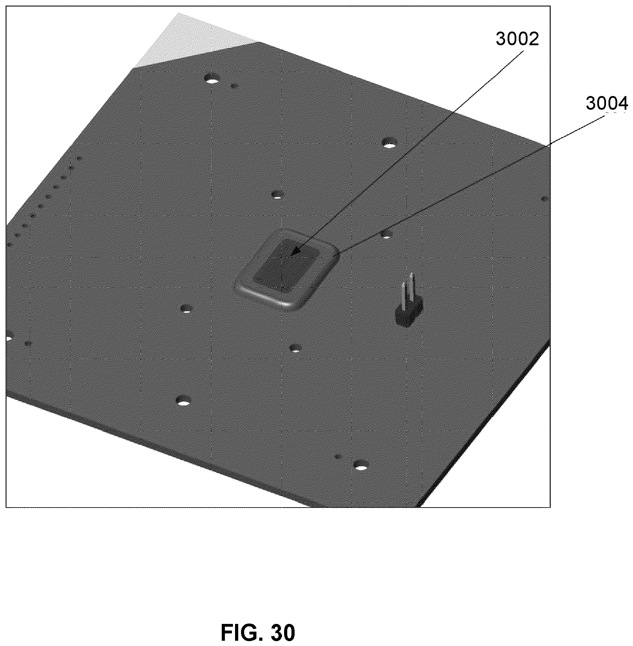

Figure 30 is a diagram illustrating an embodiment of encapsulating wire bonds together with one or more flow channel components in a single manufacturing step. For example, rather than encapsulating wire bonds between a nanopore chip and a circuit board in a separate step from securing one or more flow channel components to the nanopore chip, a single application of encapsulant 3004 is utilized to both encapsulate the wire bonds and couple/secure the one or more flow channel components to the chip. In some embodiments, without first encapsulating wire bonds, flow channel component 3002 is placed on the nanopore chip. Then, a single application of encapsulant (e.g., adhesive) is applied to simultaneously encapsulate the wire bonds and seal the perimeter of flow channel component 3002 to the nanopore based sequencing chip. By combining two manufacturing steps into one encapsulation step, manufacturing becomes more efficient and simplified. Additionally, the chip encroachment restriction on the application of the encapsulant is eliminated because the flow channel component is already placed on the chip and limits the flow of the encapsulant on undesired portions of the chip. Examples of flow channel component 3002 include various components placed on a nanopore cell to form a chamber/flow channel over the sensors of the chip. For example, various gaskets, plates, and reference electrodes described in various embodiments herein may be included in flow channel component 3002. In some embodiments, flow channel component 3002 includes molded flow channel component 2302 of Figure 23.

### Fluidic Interfaces

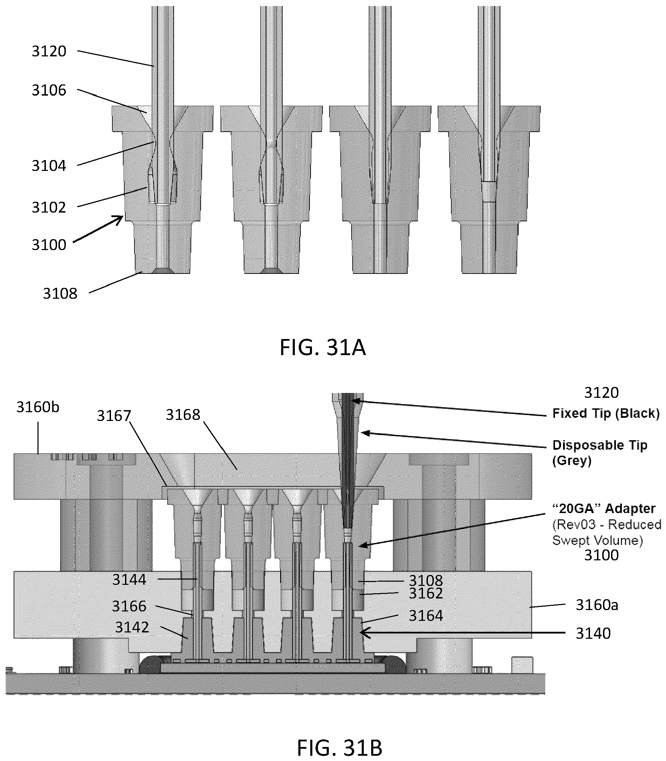

Figures 31A and 31B illustrate an embodiment of an adapter 3100 that helps form a fluidic seal between the flow cell 3140 and the dispense tip 3120. The adapter 3100 can have a lumen 3102 for receiving the dispense tip 3120 and providing a fluidic connection with the flow cell 3140. The lumen 3102 can have a constricted region 3104 with a diameter that is smaller than the diameter of the other portions of the lumen 3102. The 3104 constricted region 3104 is designed to form a seal with the dispense tip 3120 when the dispense tip 3120 is inserted into the lumen 3102 of the adapter 3100. The lumen 3102 can have an inlet 3106 for receiving the dispense tip 3120 that is conical or tapered outwards such that the inlet diameter is greater than the internal diameter of the lumen 3102. A conical or tapered inlet 3106 facilitates both insertion and alignment of the dispense tip 3120 into the lumen 3102 of the adapter 3100. The outlet 3108 of the adapter 3100 can be sized and shaped to be complementary to a receptacle 3162 in the flow cell backer 3160a.

The adapter 3102 can be made of a material with a durometer that allows the constricted region 3104 of the lumen 3102 to deform and conform to the dispense tip 3120 to provide a fluidic seal, while also being sufficiently rigid to not undergo excessive deformation when assembled between the flow cell 3140 and flow cell backer 3160a, 3160b, and subjected to various pressures (i.e., from mechanical compression between the flow cell and flow cell cover, and from fluid pressurization during use), which could result in leaks. In some embodiments, the Shore durometer can be between about 40 to 80A. In some embodiments, the durometer can be between about 50 to 70A. In some embodiments, the durometer can be between about 30 to 90A.

One embodiment of a disposable consumable device that can be used with the sequencer is formed by the assembly of the flow cell 3140 over the nanopore chip, with the adapter 3100 and flow cell backer 3160a, 3160b. The flow cell 3140 can have one or more bosses 3142 for each fluid channel the flow cell 3140 forms over the nanopore chip array. The bosses 3142 can fit into corresponding receptacles 3164 in the flow cell backer 3160a. In some embodiments, a cannula 3144 can extend from the bosses 3142 and through a channel 3166 in the flow cell backer 3160a that connects the two sets of receptacles 3162, 3164. The cannula 3144 can extend into the receptacle 3162 in which the adapter 3100 is inserted. The adapter 3100 can be inserted into the receptacle 3162 such that the cannula 3144 extends into the lumen 3102 of the adapter 3100. For each fluid channel in the flow cell 3140 that is to be used, an adapter 3100 can be inserted into the corresponding receptacle 3164. A second portion of the flow cell backer 3160b can then be placed over the adapters 3100 to secure the adapters 3100 in place over the flow cell 3140. The second portion of the flow cell backer 3160b can have a recess 3167 for receiving the adapters 3100 and an opening 3168 that provides access to the inlet 3106 of the adapter 3100.

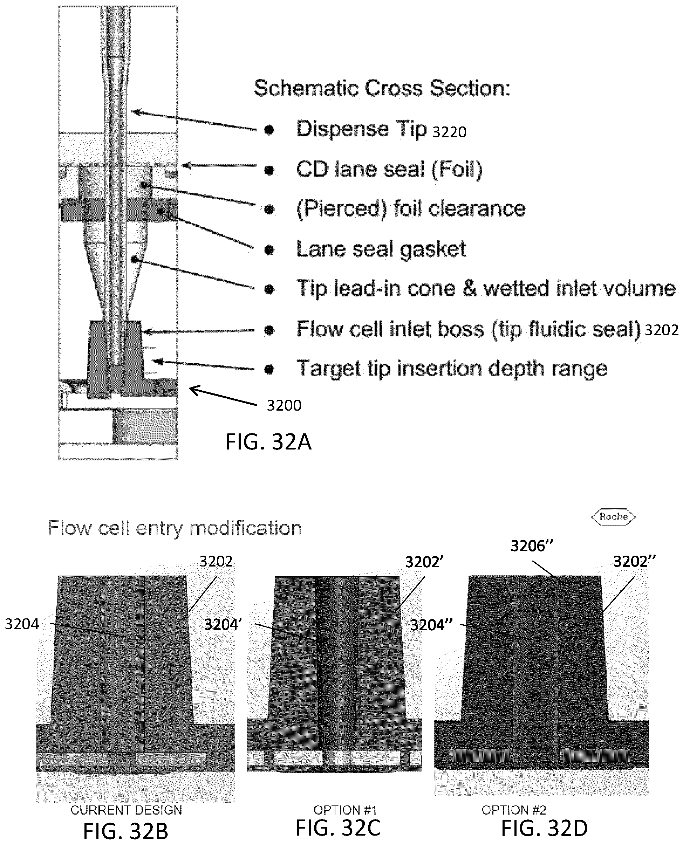

Figures 32A-32I illustrate various embodiments of direct injection fluidic interfaces that can be integrated into the flow cell and that eliminate the use of an adapter. For example, FIGS. 32A and 32B illustrate one embodiment of a direct injection flow cell 3200. The flow cell 3200 can have an inlet boss 3202 for each flow channel in the flow cell. The inlet boss 3202 can have a lumen 3204 in fluid communication with the fluid channels over the nanopore chip array. In some embodiments, as shown in FIG. 32B, the lumen 3204 can have a constant diameter that is slightly less than the diameter of the dispense tip 3220 so that a tight fluidic seal can be formed between the dispense tip 3220 and inlet boss 3202 when the dispense tip 3220 is inserted into the lumen 3204. As described above in connection with FIGS. 31A and 31B, the durometer for the inlet boss 3202 can be soft enough to allow the inlet boss 3202 to deform and conform against the dispense tip 3220, but also hard enough to maintain the fluidic seal when the flow channels are pressurized with fluids.

FIG. 32C illustrates another embodiment of an inlet boss 3202'. In this embodiment, the inlet boss 3202' can have a tapered lumen 3204' having a wider diameter at the inlet that receives the dispose tip and a narrower diameter at the outlet that leads to the interior of the flow cell. Such a configuration facilitates insertion of the dispense tip into the inlet boss and formation of a fluid tight seal around the dispense tip. FIG. 32D illustrates yet another embodiment of an inlet boss 3202" with a lumen 3204" with a chamfered inlet opening 3206‴ with a wider diameter than the lumen 3204". Similar to the tapered lumen embodiment described above, the wider diameter at the inlet opening 3206" facilitates insertion of the dispense tip into the lumen 3204", while the narrower portion of the lumen 3204" forms a tight fluidic seal with the dispense tip.

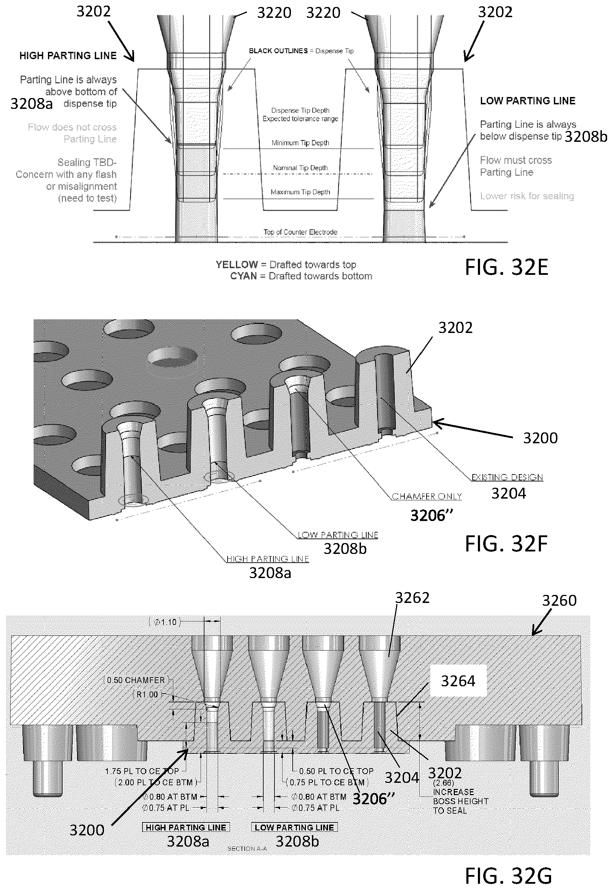

FIG. 32E illustrates a cross-section of an inlet boss 3202 with a high parting line 3208a and an inlet boss 3202 with a low parting line 3208b. FIGS. 32F and 32G illustrate a cross-section of an inlet boss 3202 with a high parting line 3208a, an inlet boss 3202 with a low parting line 3208b, an inlet boss 3202" with a chamfered inlet opening 3206" and a constant diameter lumen 3204", and an inlet boss 3202 with a constant diameter lumen 3204. FIG. 32G also illustrates a flow cell backer 3260 placed directly over the flow cell 3240 as shown in FIG. 32F. In the inlet boss 3202 with the high parting line 3208a, the dispense tip 3220 is inserted past the high parting line 3208a, such that the depth of the dispense tip 3220 is below the high parting line 3208a when the dispense tip 3220 is fully inserted into the lumen of the inlet boss 3202. In the inlet boss 3202 with the low parting line 3208b, the dispense tip 3220 is not inserted past the low parting line 3208b, and instead, the depth of the dispense tip 3220 is above low parting line 3208b when the dispense tip 3220 is fully inserted into the lumen of the inlet boss 3202. With a high parting line, the dispense tip crosses the parting line with full insertion, which may be riskier for sealing. The benefit, however, is that the fluid flow could be cleaner because the fluid does not need to cross the parting line. With a low parting line, the dispense tip does not cross the parting line, so the seal is more reliable. However, the fluid flow must now cross the parting line and may be not as ideal as a result.

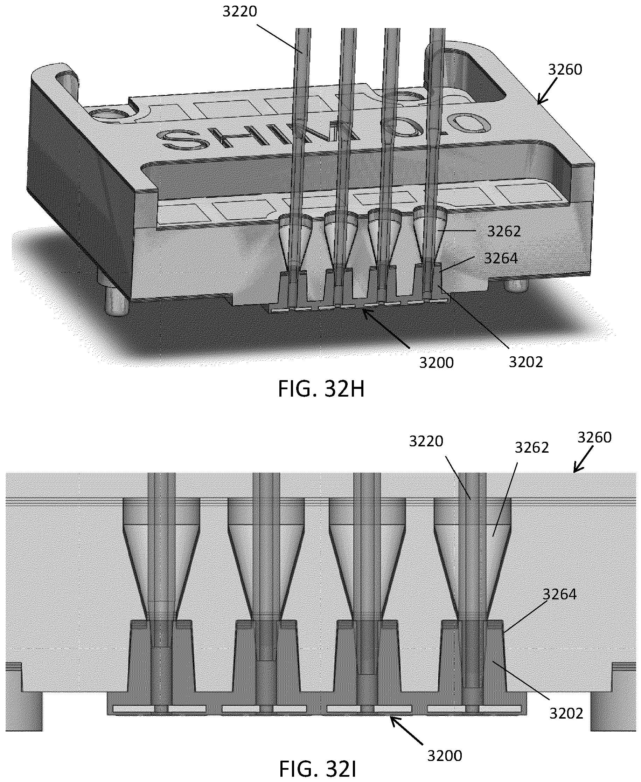

As shown in FIGS. 32F-32I, the flow cell backer 3260 can have receptacles 3264 sized and shaped for receiving the inlet bosses 3202 and connected receptacles 3262 for receiving the dispense tip 3220. The receptacles 3262 for receiving the dispense tip 3220 can be conical or funnel shaped to help guide the dispense tip 3220 into the lumen of the inlet boss 3202. The diameter of inlet of the receptacles 3262 for receiving the dispense tip 3220 is greater than the diameter of the inlet opening of the inlet boss 3202, and the diameter of the outlet of the receptacles 3262 is greater than or equal to the diameter of the inlet opening of the inlet boss 3202. In FIGS. 32H and 32I, the dispense tip 3220 is shown inserted into the lumen of the inlet boss 3202 of the flow channel to different depths. As shown, the diameter of the lumen of the inlet boss 3202 is less than the diameter of the dispense tip 3220. The inlet boss 3202 can be made of a material that deforms and/or compresses to form a fluidic seal against the dispense tip 3220.

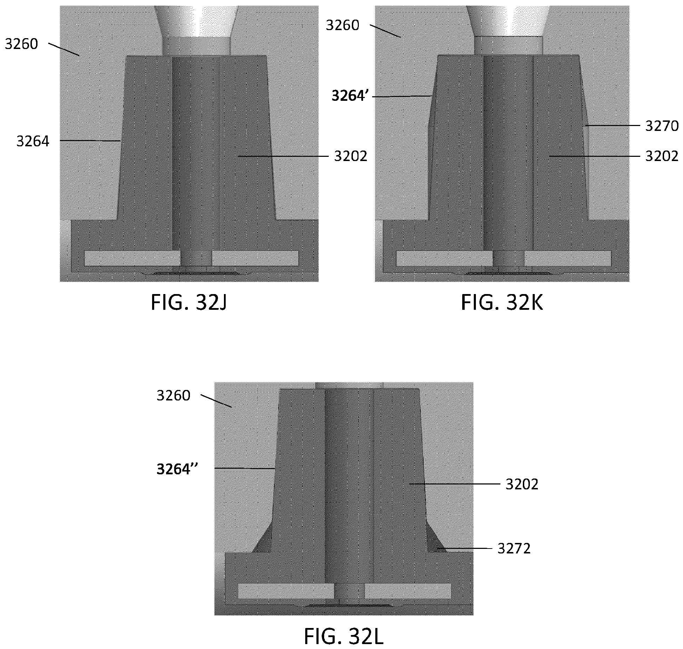

Figures 32J-32L illustrate additional embodiments of inlet boss receptacles 3264 that are designed to reduce overcompression of the inlet bosses 3202. Radial overcompression of the inlet boss 3202 can cause excessive deformation of the walls and lumen of the inlet boss 3202 such that an inadequate seal is formed around the dispense tip, which can result in fluid leakage. By oversizing the receptacle relative to the inlet boss, a space between the receptacle and inlet boss can be provided that accommodates deformation of the inlet boss upon insertion, thereby preserving the lumen of the lumen boss. Without the extra space, deformation of the inlet boss would result in inward deformation that would tend to collapse the lumen of the inlet boss. For example, as shown in FIG. 32J, the receptacles 3264 of the flow cell backer 3260 can be made larger, by increasing the diameter of the receptacles for example, in order to apply less radial compressive force to the inlet bosses 3202. In some embodiments, the diameter of the receptacles is greater than the diameter of the inlet bosses. For example, the diameter of the receptacles can be about 25 to 100 um greater, or about 10 to 150 um greater, or about 10, 15, 20, 25, 30, 35, 40, 45, 50, 55, 60, 65, 70, 75, 80, 85, 90, 95, 100, 105, 110, 115, 120, 125, 130, 135, 140, 145, or 150 um greater, or at least about 10, 15, 20, 25, 30, 35, 40, 45, 50, 55, 60, 65, 70, 75, 80, 85, 90, 95, 100, 105, 110, 115, 120, 125, 130, 135, 140, 145, or 150 um greater, or at most 10, 15, 20, 25, 30, 35, 40, 45, 50, 55, 60, 65, 70, 75, 80, 85, 90, 95, 100, 105, 110, 115, 120, 125, 130, 135, 140, 145, or 150 um greater than the diameter of the inlet bosses. In some embodiments, the shape and diameter of the receptacles 3264 and the inlet bosses 3202 are equal or about equal. In some embodiments, the diameter of the receptacles 3264 is between about 0 to 1%, 0 to 2%, 0 to 3%, 0 to 4%, 0 to 5%, 0 to 6%, 0 to 7%, 0 to 8%, 0 to 9%, 0 to 10%, 0 to 11%, 0 to 12%, 0 to 13%, 0 to 14%, or 0 to 15% larger than the diameter of the inlet bosses 3202. In other embodiments, the diameter of the receptacles 3264 is between about 1 to 2%, 1 to 3%, 1 to 4%, 1 to 5%, 1 to 6%, 1 to 7%, 1 to 8%, 1 to 9%, 1 to 10%, 1 to 11%, 1 to 12%, 1 to 13%, 1 to 14%, or 1 to 15% larger than the diameter of the inlet bosses 3202. In some embodiments, the degree or magnitude of oversizing of the receptacle depends of the hardness/softness (i.e. durometer) of the inlet boss material. A softer material would tend to undergo more deformation, and therefore, the receptacle can be oversized to a larger degree. Conversely, an inlet boss made of a harder material would tend to deform less, and therefore, a smaller degree of oversizing of the receptacle would be sufficient to prevent collapse of the lumen.

FIG. 32K illustrates another embodiment of a receptacle 3264' that has a bowed sidewall that leaves a gap 3270 between the inlet boss 3202 and the sidewall receptacle 3264'. The gap 3270 prevents undue pressure from being applied to inlet boss 3202. As shown, the gap 3270 can have a maximum width at an intermediate location along the inlet boss 3270 and receptacle 3264' and taper away to about zero at the top and bottom of the inlet boss 3202 and receptacle 3264'. This configuration allows the flow cell cover to be precisely aligned with the flow cell cover, while also providing a gap to relieve compression to the inlet bosses. Ins some embodiments, the bowed wall can be curved. In other embodiments, the bowed wall can be formed from two or more angled surfaces. In other embodiments, the receptacle can have straight walls (in a cross-sectional view) while the outer wall of the inlet boss can have a bowed surface to form the gap. In some embodiments, a gap between the receptacle and the inlet wall can be provided along an intermediate portion of the inlet boss and receptacle by removing material around an intermediate portion of the receptacle and/or inlet boss.

FIG. 32L illustrates yet another embodiment of a receptacle 3264" with a chamfered edge 3272 around the opening of the receptacle 3264". The chamfered edge 3272 provides a gap that functions to relieve compression, particularly around the base of the inlet boss 3202. In some embodiments, the receptacle and or inlet boss can be modified using any combination of the features recited herein to reduce overcompression.

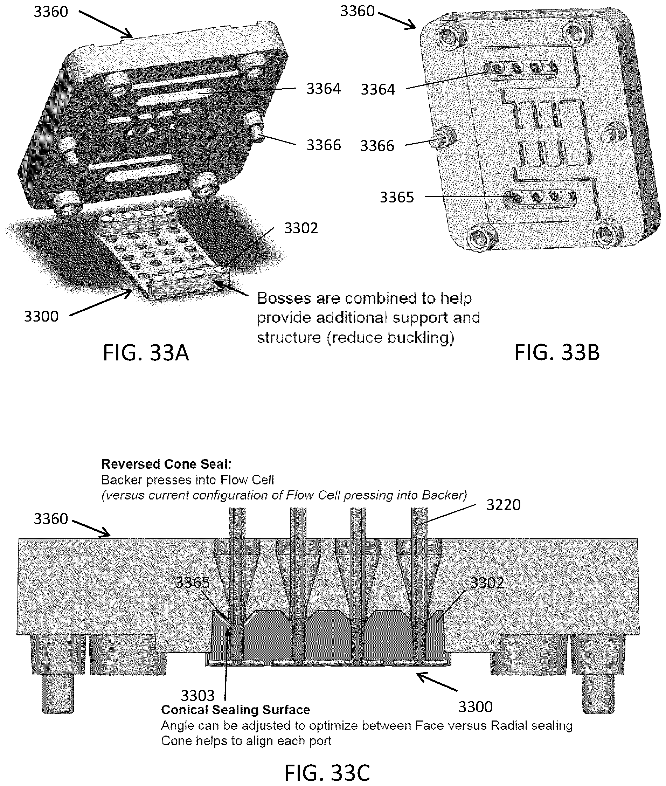

Figures 33A-33C illustrate additional embodiments of fluidic interfaces that can be integrated into the flow cell 3300. In this embodiment, the inlet bosses 3302 can be combined together into a single structure, such as a wall structure with a plurality of receptacles. Combining the inlet bosses 3302 into a single structure provides additional support to the inlet bosses and reduces the likelihood of buckling when the dispense tip 3220 is inserted into the inlet boss 3302. In some embodiments, the inlet bosses 3302 can have conical sealing surfaces 3303 that are similar to the chamfer inlet opening as described above, except that the conical sealing surfaces may be larger to take advantage of the additional material of the wall structure between each receptacle/fluidic interface. The angle of the conical sealing surface can be adjusted to increase or decrease a radially directed sealing force that develops when the flow cell backer 3360 is pressed against the flow cell 3300. In some embodiments, the angle of the conical sealing surface can be between about 30 to 60 degrees relative to an axis that extends through the lumen of the flow cell 3300. In other embodiments, the angle of the conical sealing surface can be relatively shallow and can be between about 60 to 89 degrees, or between about 70 to 89 degrees, or between about 80 to 89 degrees, or between about 85 to 89 degrees, or between about 86 to 88 degrees, relative to an axis that extends through the lumen of the flow cell 3300. The conical sealing surface feature can be used in other embodiments, such as the individual bosses shown in FIGS. 31B to 32L, for example. The flow cell backer 3360 can have receptacles 3364 with a complementary shape to the inlet boss 3302 structure, including complementary conical sealing surfaces 3365 that are configured to abut against the conical sealing surfaces 3303 of the inlet bosses 3302.

The flow cell backer 3360 can have one or more alignment pins 3366 and one or more fastening mechanisms 3368, such as screw holes located at the corners of the flow cell backer 3360, that allow the flow cell backer 3360 to be fastened securely and evenly over the flow cell 3300 and to a substrate, such as a printed circuit board (PCB). Other fastening mechanisms include snaps or clips.

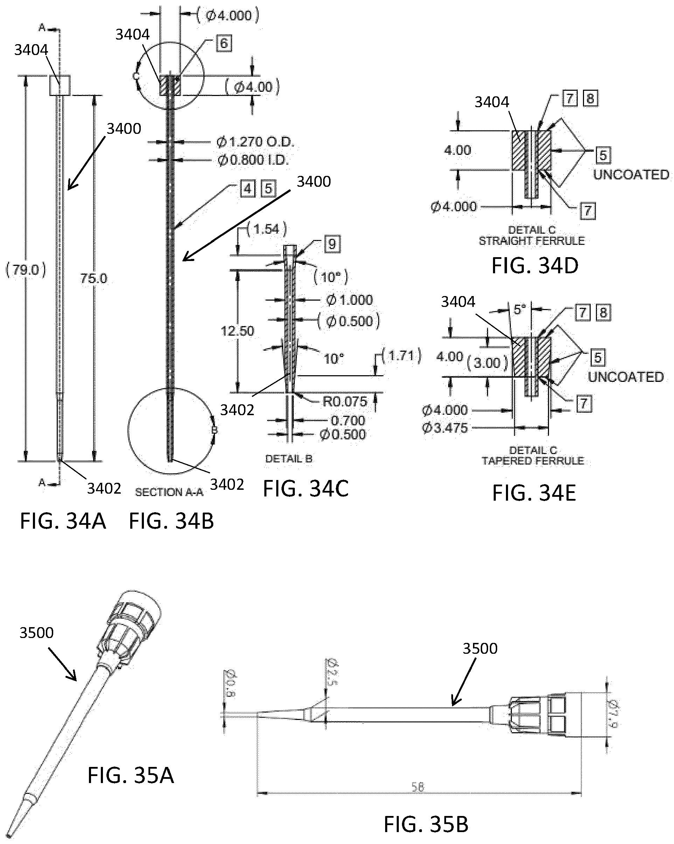

Figures 34A-34E illustrate an embodiment of a dispense tip 3400 that can be inserted into the fluidic interface of the flow cell to form a fluidic seal. The dispense tip 3400 can be made of stainless steel or another metal or metal alloy and can be coated with a low friction, hydrophobic material, such as a fluoropolymer (i.e., polytetrafluoroethylene (PTFE) or fluorinated ethylene propylene (FEP)). Both the outer surface and the interior surface of the dispense tip 3400 can be coated. In some embodiments, the coating is at least 1, 2, 3, 4, or 5 um thick. Use of metal for the dispense tip over other materials, such as plastic, provides the dispense tip with increased structural strength and allows the dispense tip to be used repeatedly before needing to be replaced. Use of metal also allows the dispense tip to function as a probe, such as a liquid level probe using capacitive sensing. The length of the dispense tip 3400 can be sufficiently long to ensure that the dispense tip can reach the bottom of the reagent reservoirs that are used. For example, the length can be between about 20 to 200 mm, or between about 40 to 100 mm, or between about 60 to 80 mm, or at least 20, 30, 40, 50, 60, 70, 80, 90, or 100 mm in length. In some embodiments, the volume of the lumen of the dispense tip 3400 can be sufficiently large to ensure that the entire reagent and/or sample volume that is to be dispensed can be held within the lumen of the dispense tip 3400 in order to prevent the reagents and/or sample from being aspirated into the tubing, which may cause the waste of precious reagents. For example, the swept volume of the dispense tip 3400 can be between 10 and 100 ul, or at least 10, 20, 30, 40, 50, 60, 70, 80, 90, or 100 ul. The outer diameter of the dispense tip 3400 can be slightly greater than the diameter of the lumen of the inlet boss of the flow cell. The tip 3402 of dispense tip 3400 may be gently tapered to facilitate insertion into the lumen of the inlet boss and formation of a fluidic seal. In some embodiments, the taper can be between about 5 to 30 degrees, or be about 5, 10, 15, 20, 25, or 30 degrees. A ferrule 3404 can be placed on the proximal end of dispense tip in order to provide an attachment feature that can be used to attached the dispense tip to the dispensing head of the instrument. In addition, the ferrule also acts as: (1) a fluidic sealing surface between the instrument and the dispense tip, and an electrical connection between the instrument and the dispense tip so that the dispense tip can function as a sensor in a capacitive liquid level detection circuit. The ferrule may be uncoated and can be either straight or slightly tapered. In some embodiments, the dispense tip 3400 may also be used to puncture a seal covering the inlet boss.

Figures 35A and 35B illustrate an embodiment of a disposable dispense tip 3500 that can be made of a plastic material, for example. The swept volume and/or the dimensions of the disposable dispense tip 3500 can be the same or similar to that described above for the reusable dispense tip 3400. In some embodiments, the plastic material can be made of a conductive polymer or contain an embedded electrode in order for the dispense tip to function as a probe, such as a capacitive liquid level probe. In some embodiments, the disposable dispense tip 3500 can be coated with a hydrophobic material, such as a fluoropolymer, similar to that as described above for metal dispense tips.

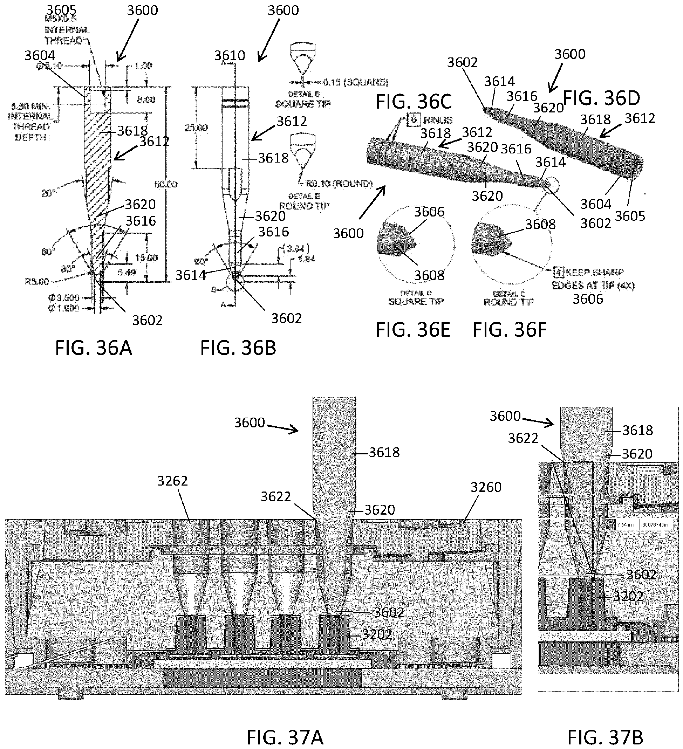

Figures 36A-36F illustrate an embodiment of a piercing tool 3600 that can be used to create an opening in a seal covering the fluidic interface. In some embodiments, the piercing tool 3600 can be made of metal or metal alloy, such as stainless steel. In other embodiments, the piercing tool 3600 can be made of a polymer or ceramic material. The piercing tool 3600 can have a distal end 3602 with a sharp tip for piecing the seal and a proximal end 3604 with an attachment feature 3605 or mechanism, such as internal screw threads, for attaching the piercing tool 3600 to an actuator.

In some embodiments, the distal end 3602 can have a plurality of sharp cutting edges 3606 and cutting faces 3608 that can be angled between about 15 degrees to about 75 degrees from a longitudinal axis 3610 that extends through an elongate shaft 3612 of the piercing tool 3600. The angle of the cutting edges 3606 and cutting faces 3608 can be set along with the seal material and thickness such that the force required to pierce the seal can be between about 5 to 10 N. In some embodiments, the distal end 3602 can have a two stage taper, with the first taper as described above with respect to the cutting edges 3606 and cutting faces 3608 located at the very end of the piercing tool. In some embodiments, a second tapering section 3614 can extend proximally from the cutting edges 3606 and cutting faces 3608 at an angle that is more acute than the angle of the cutting edges 3606 and cutting faces 3608. For example, the second tapering section 3614 can have an angle that is about 5 to 45 degrees less the angle of the cutting edges 3606 and cutting faces 3608. The second taper is designed to dilate the opening in the foil during the piercing step to a maximum diameter and minimize any `foil flaps' that might interfere with the dispensing of liquid from the dispense tip. To achieve these functions, the second taper can have a maximum diameter that is greater than the diameter of the receptacle to ensure that the opening in the foil is created all the way to the outer edges of the receptacle.

In some embodiments, the piercing tool 3600 can have an elongate shaft 3612 with at least two sections, a distal section 3616 and a proximal section 3618, of different diameters, with the distal section 3616 having a smaller diameter than the proximal section 3618. A third tapering section 3620 can provide a transition between distal section 3616 and the proximal section 3618. In some embodiments, the third tapering section 3620 can have an angle between about 10 and 60 degrees. In some embodiments, the third tapering section 3620 has an angle that is equal to or less than the second tapering section 3614.

Figures 37A-37B illustrate a method of piercing the seal of the flow cell and inserting the dispense tip into the fluidic interface in a manner that does not introduce air bubbles into the flow cell. In some embodiments, the diameter of the proximal section 3618 can be greater than the opening of the receptacle 3262 of the consumable device backer 3260 into which the piercing device 3600 is inserted, such that a location along the third tapering section 3620 has a diameter equal to the diameter of the opening and functions as a stop to limit further advancement of the piercing tool 3600 into the consumable device. In some embodiments, the piercing tool is driven to a set, target (i.e. predetermined) force to ensure that the tip of the piercing tool bottoms out against the stop in order to create the largest opening possible. In contrast, driving or inserting the piercing tool to a set depth past the seal, may create different sized openings in the foil due to positioning tolerances of the tip of the piercing tool and to differences in the conical shape of the piercing tool. In some embodiments, the length of the piecing tool 3600 from the distal end 3602 to the stop location 3622 on the third tapering section 3620 can be approximately equal to or less than the distance between the opening in the consumable device backer 3260 to the top of the inlet boss 3202 of the flow cell interface, in order to prevent the piercing tool 3600 from being inserted into the inlet boss 3202 of the flow cell interface.

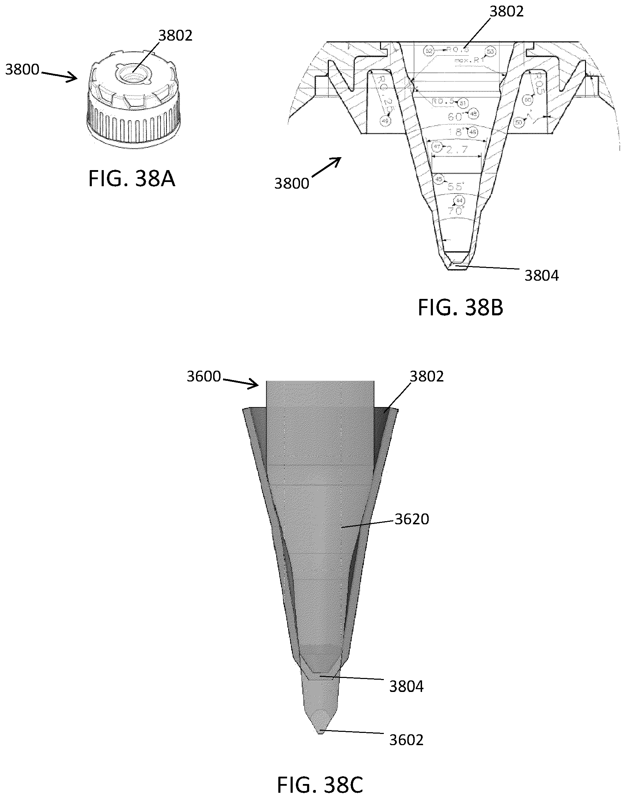

FIGS. 38A-38C illustrate a reagent bottle cap 3800 that can be also pierced by the piercing tool 3600. In some embodiments, the reagent bottle cap 3800 can have a receptacle 3802 for receiving the piercing tool 3600. In some embodiments, the receptacle 3802 can be tapered and can be wider at the opening and narrower at the bottom 3804. The piercing tool 3600 can have a length and diameter that allows the distal end 3602 of the piercing tool 3600 to be inserted into the receptacle far enough to pierce the bottom 3804 of the receptacle 3802. In some embodiments, one of the tapering portions of the piercing tool 3600, such as the third tapering portion 3620 has a diameter at a portion of the third tapering portion 3620 that is equal to a diameter of an intermediate portion of the tapered receptacle 3802. The portion of the third tapering portion 3620 with a matching diameter as the intermediate portion of the tapered receptacle functions as a stop, and the length of the piercing tool 3600 from the portion of the third tapering portion 3620 to the distal end 3602 of the piercing tool 3600 is greater than the length between the intermediate portion of tapered receptacle 3802 to the bottom of the tapered receptacle 3802 so that the piercing tool 3600 penetrates through the bottom 3804 of the tapered receptacle 3802 when fully inserted into the receptacle 3802.

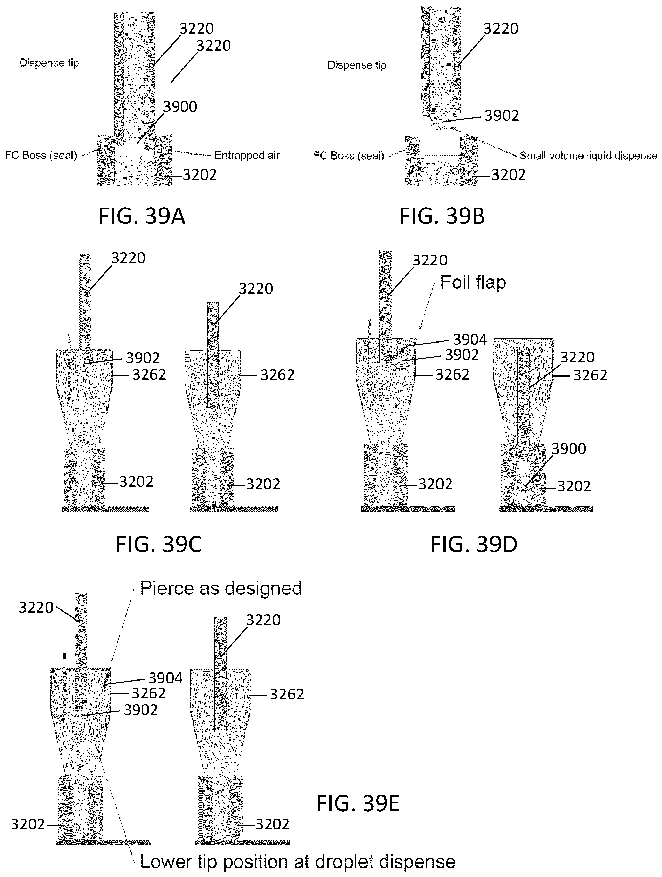

FIGS. 39A-39E illustrate a system and method of making a reliable fluid to fluid connection between the fluid in the dispense tip 3220 and the fluid in the receptacle 3262 of the consumable device backer, thereby preventing the inadvertent introduction of a bubble into the fluidics. As shown in FIG. 39A, the dispense tip 3220 can have lumen that his filled with a fluid ready to be dispensed. However, a small amount of gas 3900 (i.e. air) can be trapped at the distal end of the lumen of the dispense tip 3220. If the dispense tip 3220 is inserted into the fluid in receptacle 3262 and inserted into the inlet boss 3202 when gas is trapped in the dispense tip 3220, gas 3900 can be introduced into the fluidics when fluid is dispensed from the dispense tip 3220.

As shown in FIGS. 39B and 39C, a small amount of fluid can be partially dispensed from the dispense tip so that a partial droplet 3902 of fluid can extend from the distal end of the dispense tip 3220. This ensures that no gas is trapped within the lumen of the dispense tip 3220 so that a fluid to fluid connection can be properly made between the droplet 3902 and the fluid in the receptacle 3262 when the dispense tip 3220 is inserted into the receptacle 3262.

As shown in FIG. 39D, a covering, such as a foil or plastic sheet, can be used to seal the receptacles 3262 before use. A piercing tool as described herein can be used to pierce the covering. In some embodiments, a flap 3904 (i.e., a foil or plastic flap) can be formed from the covering after the covering has been pierced by the piercing tool. In some embodiments, this flap 3904 can extend into the lumen of the receptacle 3262 and can make contact with the distal end of the dispense tip 3220 when the dispense tip 3220 is inserted into the receptacle 3262. If fluid has been partially dispensed from the dispense tip 3220 as a partial droplet 3902 before the distal end of the dispense tip 3902 has been inserted past the flap 3904, then the droplet 3902 may be wicked from the distal end of the dispense tip 3220, which can result in a small amount of gas 3900 at the distal end of the dispense tip 3220 as shown in FIG. 39A. If the dispense tip 3220 is then inserted into the fluid in the receptacle 3202, the gas 3900 in the dispense tip 3220 can be transferred into the inlet boss 3202 and into the fluidic system. In some embodiments, to reduce the likelihood of wicking, the flaps 3904 are pushed against the sidewalls of the receptacle 3262 by a proximal portion of the piercing tool that can at least partially match the geometry of the upper portion of the receptacle adjacent to the foil cover.

As shown in FIG. 39E, to reduce the risk of wicking away the droplet 3902 from the dispense tip 3220, the distal end of the dispense tip 3220 can be inserted past the flap 3904 before forming the partial droplet 3902 at the distal end of the dispense tip 3220. For example, the distal end of the dispense tip 3220 can be inserted into the receptacle 3262 past the flap 3904. Then fluid can be partially dispensed from the distal end of the dispense tip 3220 to expel any trapped gas in the lumen and to form a partial droplet 3902 at the distal end of the dispense tip 3220. The dispense tip 3220 with the partial droplet 3902 can then be lowered to make the fluid to fluid connection with the fluid in the receptacle.

Although the foregoing embodiments have been described in some detail for purposes of clarity of understanding, the invention is not limited to the details provided. There are many alternative ways of implementing the invention. The disclosed embodiments are illustrative and not restrictive.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

Although various illustrative embodiments are described above, any of a number of changes may be made to various embodiments without departing from the scope of the invention as defined by the claims. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of the invention. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

## Claims

1. A consumable device for use with a sequencing system, the consumable device comprising:
a sequencing chip, the sequencing chip comprising a plurality of wells, whereby each well comprises a working electrode and is a nanopore based sequencing cell;
a flow cell comprising:
at least one flow channel, wherein the flow cell is configured to be disposed over the sequencing chip (602, 802) such that the at least one flow channel (1608, 1708) is disposed over the plurality of wells of the sequencing chip (602, 802);
at least one inlet boss (3202) having a lumen (3204) in fluid communication with the at least one flow channel (1608, 1708) and
at least one counter electrode (812, 1218, 1906) disposed over at least a portion of the at least one flow channel (1608, 1708), and **characterized by**
a flow cell cover comprising:
at least one inlet boss receptacle (3264) for receiving the at least one inlet boss (3202) of the flow cell; and
at least one dispense tip receptacle (3262) configured to receive a dispense tip (3220), wherein the at least one dispense tip receptacle (3262) is in fluid communication with the at least one inlet boss receptacle (3262).

2. The consumable device of claim 1, wherein the sequencing chip (602, 802) comprises at least about 8 million wells.

3. The consumable device of claim 1, wherein the at least one dispense tip (3220) receptacle is funnel shaped.

4. The consumable device of claim 1, wherein the at least one dispense tip receptacle (3262) is connected to the at least one inlet boss receptacle via a hole or lumen sized to pass the dispense tip (3220). .

5. The consumable device of claim 1, wherein the lumen of the at least one inlet boss (3202) has a constant diameter.

6. The consumable device of claim 1, wherein the lumen of the at least one inlet boss (3202) is tapered.

7. The consumable device of claim 1, wherein the lumen of the at least one inlet boss (3202) comprises a chamfered inlet opening.

8. The consumable device of claim 1, wherein the at least one inlet boss (3202) is made of a deformable material capable of conforming to the dispense tip.

9. The consumable device of claim 1, wherein the at least one inlet boss receptacle (3264) has a bowed surface configured to form a gap (3270) with the at least one inlet boss (3202) when the at least one inlet boss (3202) is inserted into the at least one inlet boss receptacle (3264).

10. The consumable device of claim 1, wherein the at least one inlet boss receptacle (3264) has a chamfered opening.

11. The consumable device of claim 1, wherein the at least one inlet boss (3202) comprises a plurality of inlet lumens that are combined into a single inlet boss (3202).

12. The consumable device of claim 11, wherein each inlet lumen has a conical opening, wherein the at least one dispense tip receptacles (3262) comprises a plurality of dispense tip receptacles (3262) in fluid communication with a single inlet boss receptacle (3264) configured to receive the single inlet boss (3202), wherein the single inlet boss receptacle (3264) comprises a plurality of conical sealing surfaces configured to mate with each conical opening of each inlet lumen.

13. The consumable device of claim 1, wherein the at least one dispense tip receptacle (3262) is sealed with a pierceable material.

14. A method of delivering fluid to a consumable device for sequencing, the method comprising:
inserting a piercing tool (3600) through a seal that is covering a dispense tip receptacle (3262) of a consumable device, the consumable device comprising:
a sequencing chip (602, 802), the sequencing chip comprising a plurality of wells, wherein each well comprises a working electrode and is a nanopore based sequencing cell;
a flow cell comprising:
at least one flow channel (1608, 1708), wherein the flow cell is configured to be disposed over the sequencing chip (602, 802) such that the at least one flow channel is disposed over the plurality of wells of the sequencing chip (602, 802);
at least one inlet boss (3202) having a lumen in fluid communication with the at least one flow channel (1608, 1708); and
at least one counter electrode disposed over at least a portion of the at least one channel (1608, 1708); and being **characterized by** that the consumable device comprises
a flow cell cover comprising:
at least one inlet boss receptacle (3264) for receiving the at least one inlet boss (3202) of the flow cell; and
at least one dispense tip receptacle (3262) configured to receive a dispense tip (3220), wherein the at least one dispense tip receptacle (3262) is in fluid communication with the at least one inlet boss receptacle receptacle (3264);
removing the piercing tool (3600) from the dispense tip receptacle (3262) and leaving one or more seal fragments that extend into the dispense tip receptacle (3262);
inserting a distal end of a dispense tip (3220) past the one or more seal fragments;
after the step of inserting the distal end of the dispense tip (3220) past the one or more seal fragments, partially dispensing a first fluid from the distal end of the dispense tip (3220) so that the first fluid extends distally from the distal end as a partial droplet; and
advancing the dispense tip (3220) so that the partial droplet makes a liquid-liquid connection with a second fluid in the dispense tip receptacle (3262).

15. The method of claim 14, further comprising advancing the distal end of the dispense tip (3220) into the lumen of the at least one inlet boss receptacle (3264) to make a fluid tight seal between the dispense tip and the at least one inlet boss (3202).

16. The method of claim 15, further comprising delivering fluid through the dispense tip (3220) into the at least one flow channe (1608, 1708) .

17. The method of claim 14, further comprising piercing a seal of a reagent reservoir with the piercing tool (3600), wherein the first fluid is stored in the reagent reservoir.

18. The method of claim 17, wherein the seal is a pierceable cap.

19. The method of claim 17, wherein the reagent reservoir is selected from the group consisting of a bottle and a trough.

## Patentansprüche

1. Einwegvorrichtung zur Verwendung mit einem Sequenzierungssystem, wobei die Einwegvorrichtung Folgendes umfasst:
einen Sequenzierungschip, wobei der Sequenzierungschip eine Vielzahl von Wells umfasst, wobei jedes Well eine Arbeitselektrode umfasst und eine nanoporenbasierte Sequenzierungszelle ist;
eine Durchflusszelle, umfassend:
mindestens einen Durchflusskanal, wobei die Durchflusszelle dafür ausgebildet ist, so über dem Sequenzierungschip (602, 802) angeordnet zu sein, dass der mindestens eine Durchflusskanal (1608, 1708) über der Vielzahl von Wells des Sequenzierungschips (602, 802) angeordnet ist;
mindestens einen Einlasshals (3202) mit einem Hohlraum (3204) in Flüssigkeitskommunikation mit dem mindestens einen Durchflusskanal (1608, 1708) und
mindestens eine Gegenelektrode (812, 1218, 1906), die über mindestens einem Abschnitt des mindestens einen Durchflusskanals (1608, 1708) angeordnet ist, und **gekennzeichnet durch** eine Durchflusszellenabdeckung, umfassend:
mindestens eine Einlasshalsaufnahme (3264) zum Aufnehmen des mindestens einen Einlasshalses (3202) der Durchflusszelle und
mindestens eine Dispensierspitzenaufnahme (3262), die zum Aufnehmen einer Dispensierspitze (3220) ausgebildet ist, wobei die mindestens eine Dispensierspitzenaufnahme (3262) in Flüssigkeitskommunikation mit der mindestens einen Einlasshalsaufnahme (3262) steht.

2. Einwegvorrichtung nach Anspruch 1, wobei der Sequenzierungschip (602, 802) mindestens etwa 8 Millionen Wells umfasst.

3. Einwegvorrichtung nach Anspruch 1, wobei die mindestens eine Dispensierspitzen-(3220)-aufnahme trichterförmig ist.

4. Einwegvorrichtung nach Anspruch 1, wobei die mindestens eine Dispensierspitzenaufnahme (3262) über ein Loch oder einen Hohlraum, das/der so dimensioniert ist, dass die Dispensierspitze (3220) hindurchgeht, mit der mindestens einen Einlasshalsaufnahme verbunden ist.

5. Einwegvorrichtung nach Anspruch 1, wobei der Hohlraum des mindestens einen Einlasshalses (3202) einen konstanten Durchmesser hat.

6. Einwegvorrichtung nach Anspruch 1, wobei der Hohlraum des mindestens einen Einlasshalses (3202) kegelförmig ist.

7. Einwegvorrichtung nach Anspruch 1, wobei der Hohlraum des mindestens einen Einlasshalses (3202) eine abgeschrägte Einlassöffnung umfasst.

8. Einwegvorrichtung nach Anspruch 1, wobei der mindestens eine Einlasshals (3202) aus einem verformbaren Material gefertigt ist, das sich der Dispensierspitze anpassen kann.

9. Einwegvorrichtung nach Anspruch 1, wobei die mindestens eine Einlasshalsaufnahme (3264) eine gewölbte Oberfläche aufweist, die zum Bilden eines Spalts (3270) mit dem mindestens einen Einlasshals (3202) ausgebildet ist, wenn der mindestens eine Einlasshals (3202) in die mindestens eine Einlasshalsaufnahme (3264) eingeführt wird.

10. Einwegvorrichtung nach Anspruch 1, wobei die mindestens eine Einlasshalsaufnahme (3264) eine abgeschrägte Öffnung aufweist.

11. Einwegvorrichtung nach Anspruch 1, wobei der mindestens eine Einlasshals (3202) eine Vielzahl von Einlasshohlräumen umfasst, die in einem einzelnen Einlasshals (3202) kombiniert sind.

12. Einwegvorrichtung nach Anspruch 11, wobei jeder Einlasshohlraum eine konische Öffnung aufweist, wobei die mindestens eine Dispensierspitzenaufnahme (3262) eine Vielzahl von Dispensierspitzenaufnahmen (3262) in Flüssigkeitskommunikation mit einer einzelnen Einlasshalsaufnahme (3264) umfasst, die zum Aufnehmen des einzelnen Einlasshalses (3202) ausgebildet ist, wobei die einzelne Einlasshalsaufnahme (3264) eine Vielzahl von konischen Dichtungsflächen umfasst, die so ausgebildet sind, dass sie in jede konische Öffnung jedes Einlasshohlraumes passen.

13. Einwegvorrichtung nach Anspruch 1, wobei die mindestens eine Dispensierspitzenaufnahme (3262) mit einem perforierbaren Material abgedichtet ist.

14. Verfahren zum Abgeben von Flüssigkeit an eine Einwegvorrichtung zum Sequenzieren, wobei das Verfahren Folgendes umfasst:
Einführen eines Perforierwerkzeugs (3600) durch eine Dichtung, die eine Dispensierspitzenaufnahme (3262) einer Einwegvorrichtung abdeckt, wobei die Einwegvorrichtung Folgendes umfasst:
einen Sequenzierungschip (602, 802), wobei der Sequenzierungschip eine Vielzahl von Wells umfasst, wobei jedes Well eine Arbeitselektrode umfasst und eine nanoporenbasierte Sequenzierungszelle ist;
eine Durchflusszelle, umfassend:
mindestens einen Durchflusskanal (1608, 1708), wobei die Durchflusszelle dafür ausgebildet ist, so über dem Sequenzierungschip (602, 802) angeordnet zu sein, dass der mindestens eine Durchflusskanal über der Vielzahl von Wells des Sequenzierungschips (602, 802) angeordnet ist;
mindestens einen Einlasshals (3202) mit einem Hohlraum in Flüssigkeitskommunikation mit dem mindestens einen Durchflusskanal (1608, 1708) und
mindestens eine Gegenelektrode, die über mindestens einem Abschnitt des mindestens einen Durchflusskanals (1608, 1708) angeordnet ist; und **dadurch gekennzeichnet, dass** die Einwegvorrichtung eine Durchflusszellenabdeckung umfasst, umfassend:
mindestens eine Einlasshalsaufnahme (3264) zum Aufnehmen des mindestens einen Einlasshalses (3202) der Durchflusszelle und
mindestens eine Dispensierspitzenaufnahme (3262), die zum Aufnehmen einer Dispensierspitze (3220) ausgebildet ist;
wobei die mindestens eine Dispensierspitzenaufnahme (3262) in Flüssigkeitskommunikation mit der mindestens einen Einlasshalsaufnahme (3264) steht;
Entfernen des Perforierwerkzeugs (3600) aus der Dispensierspitzenaufnahme (3262) und Hinterlassen eines Dichtfragments oder mehrerer Dichtfragmente, das/die sich in die Dispensierspitzenaufnahme (3262) erstreckt/erstrecken;
Einführen eines distalen Endes einer Dispensierspitze (3220) nach dem einen oder den mehreren Dichtfragment(en);
nach dem Schritt des Einführens des distalen Endes der Dispensierspitze (3220) nach dem einen oder den mehreren Dichtfragment(en), teilweises Dispensieren einer ersten Flüssigkeit aus dem distalen Ende der Dispensierspitze (3220), sodass die erste Flüssigkeit distal aus dem distalen Ende als ein partieller Tropfen fließt; und
Vorschieben der Dispensierspitze (3220), sodass der partielle Tropfen eine Flüssig-Flüssig-Verbindung mit einer zweiten Flüssigkeit in der Dispensierspitzenaufnahme (3262) herstellt.

15. Verfahren nach Anspruch 14, ferner umfassend das Vorschieben des distalen Endes der Dispensierspitze (3220) in den Hohlraum der mindestens einen Einlasshalsaufnahme (3264), um eine flüssigkeitsdichte Dichtung zwischen der Dispensierspitze und dem mindestens einen Einlasshals (3202) herzustellen.

16. Verfahren nach Anspruch 15, ferner umfassend das Abgeben von Flüssigkeit durch die Dispensierspitze (3220) in den mindestens einen Durchflusskanal (1608, 1708).

17. Verfahren nach Anspruch 14, ferner umfassend das Perforieren einer Dichtung eines Reagenzreservoirs mit dem Perforierwerkzeug (3600), wobei die erste Flüssigkeit in dem Reagenzreservoir gelagert wird.

18. Verfahren nach Anspruch 17, wobei die Dichtung eine perforierbare Kappe ist.

19. Verfahren nach Anspruch 17, wobei das Reagenzreservoir aus der Gruppe ausgewählt ist, die aus einer Flasche und einer Wanne besteht.

## Revendications

1. Dispositif consommable pour une utilisation avec un système de séquençage, le dispositif consommable comprenant :
une puce de séquençage, la puce de séquençage comprenant une pluralité de puits, moyennant quoi chaque puits comprend une électrode de travail et est une cellule de séquençage à base de nanopores ;
une cellule d'écoulement comprenant :
au moins un canal d'écoulement, dans lequel la cellule d'écoulement est conçue pour être disposée par-dessus la puce de séquençage (602, 802) de telle sorte que l'au moins un canal d'écoulement (1608, 1708) est disposé par-dessus la pluralité de puits de la puce de séquençage (602, 802) ;
au moins une protubérance d'entrée (3202) ayant une lumière (3204) en communication fluidique avec l'au moins un canal d'écoulement (1608, 1708) et
au moins une contre-électrode (812, 1218, 1906) disposée par-dessus au moins une partie de l'au moins un canal d'écoulement (1608, 1708), et **caractérisé par** un couvercle de cellule d'écoulement comprenant :
au moins un logement de protubérance d'entrée (3264) pour recevoir l'au moins une protubérance d'entrée (3202) de la cellule d'écoulement ; et
au moins un logement de pointe de distribution (3262) conçu pour recevoir une pointe de distribution (3220), l'au moins un logement de pointe de distribution (3262) étant en communication fluidique avec l'au moins un logement de protubérance d'entrée (3262).

2. Dispositif consommable selon la revendication 1, dans lequel la puce de séquençage (602, 802) comprend au moins environ 8 millions de puits.

3. Dispositif consommable selon la revendication 1, dans lequel l'au moins un logement de la pointe de distribution (3220) est en forme d'entonnoir.

4. Dispositif consommable selon la revendication 1, dans lequel l'au moins un logement de pointe de distribution (3262) est relié à l'au moins un logement de protubérance d'entrée par le biais d'un orifice ou d'une lumière dimensionnée pour laisser passer la pointe de distribution (3220).

5. Dispositif consommable selon la revendication 1, dans lequel la lumière de l'au moins une protubérance d'entrée (3202) a un diamètre constant.

6. Dispositif consommable selon la revendication 1, dans lequel la lumière de l'au moins une protubérance d'entrée (3202) est effilée.

7. Dispositif consommable selon la revendication 1, dans lequel la lumière de l'au moins une protubérance d'entrée (3202) comprend une ouverture d'entrée chanfreinée.

8. Dispositif consommable selon la revendication 1, dans lequel l'au moins une protubérance d'entrée (3202) est constituée d'un matériau déformable capable de s'adapter à la pointe de distribution.

9. Dispositif consommable selon la revendication 1, dans lequel l'au moins un logement de protubérance d'entrée (3264) a une surface incurvée conçue pour former un espace (3270) avec l'au moins une protubérance d'entrée (3202) lorsque l'au moins une protubérance d'entrée (3202) est insérée dans l'au moins un logement de protubérance d'entrée (3264).

10. Dispositif consommable selon la revendication 1, dans lequel l'au moins un logement de protubérance d'entrée (3264) a une ouverture chanfreinée.

11. Dispositif consommable selon la revendication 1, dans lequel l'au moins une protubérance d'entrée (3202) comprend une pluralité de lumières d'entrée qui sont combinées dans une unique protubérance d'entrée (3202).

12. Dispositif consommable selon la revendication 11, dans lequel chaque lumière d'entrée a une ouverture conique, dans lequel l'au moins un logement de pointe de distribution (3262) comprend une pluralité de logements de pointe de distribution (3262) en communication fluidique avec un unique logement de protubérance d'entrée (3264) conçu pour recevoir l'unique protubérance d'entrée (3202), dans lequel l'unique logement de protubérance d'entrée (3264) comprend une pluralité de surfaces d'étanchéité coniques conçues pour s'accoupler avec chaque ouverture conique de chaque lumière d'entrée.

13. Dispositif consommable selon la revendication 1, dans lequel l'au moins un logement de pointe de distribution (3262) est étanchéifié avec un matériau perçable.

14. Procédé de distribution de fluide à un dispositif consommable pour le séquençage, le procédé comprenant :
l'insertion d'un outil de perçage (3600) à travers un joint d'étanchéité qui recouvre un logement de pointe de distribution (3262) d'un dispositif consommable, le dispositif consommable comprenant :
une puce de séquençage (602, 802), la puce de séquençage comprenant une pluralité de puits, chaque puits comprenant une électrode de travail et est une cellule de séquençage à base de nanopores ;
une cellule d'écoulement comprenant :
au moins un canal d'écoulement (1608, 1708), dans lequel la cellule d'écoulement est conçue pour être disposée par-dessus la puce de séquençage (602, 802) de telle sorte que l'au moins un canal d'écoulement est disposé par-dessus la pluralité de puits de la puce de séquençage (602, 802) ;
au moins une protubérance d'entrée (3202) ayant une lumière en communication fluidique avec l'au moins un canal d'écoulement (1608, 1708) ; et
au moins une contre-électrode disposée par-dessus au moins une partie de l'au moins un canal (1608, 1708) ; et étant **caractérisé en ce que** le dispositif consommable comprend
un couvercle de cellule d'écoulement comprenant :
au moins un logement de protubérance d'entrée (3264) pour recevoir l'au moins une protubérance d'entrée (3202) de la cellule d'écoulement ; et
au moins un logement de pointe de distribution (3262) conçu pour recevoir une pointe de distribution (3220),
dans lequel l'au moins un logement de pointe de distribution (3262) est en communication fluidique avec l'au moins un logement de protubérance d'entrée (3264) ;
le retrait de l'outil de perçage (3600) du logement de pointe de distribution (3262) et le fait de laisser un ou plusieurs fragments de joint d'étanchéité qui s'étendent dans le logement de pointe de distribution (3262) ;
l'insertion d'une extrémité distale d'une pointe de distribution (3220) au-delà du ou des fragments de joint d'étanchéité ;
après l'étape d'insertion de l'extrémité distale de la pointe de distribution (3220) au-delà du ou des fragments de joint d'étanchéité, la distribution partielle d'un premier fluide depuis l'extrémité distale de la pointe de distribution (3220) de telle sorte que le premier fluide s'étend de manière distale depuis l'extrémité distale en tant que gouttelette partielle ; et
l'avancement de la pointe de distribution (3220) de telle sorte que la gouttelette partielle effectue une liaison liquide-liquide avec un second fluide dans le logement de pointe de distribution (3262).

15. Procédé selon la revendication 14, comprenant en outre l'avancement de l'extrémité distale de la pointe de distribution (3220) dans la lumière de l'au moins un logement de protubérance d'entrée (3264) pour effectuer un joint d'étanchéité étanche aux fluides entre la pointe de distribution et l'au moins une protubérance d'entrée (3202).

16. Procédé selon la revendication 15, comprenant en outre la distribution de fluide à travers la pointe de distribution (3220) dans l'au moins un canal d'écoulement (1608, 1708).

17. Procédé selon la revendication 14, comprenant en outre le perçage d'un joint d'étanchéité d'un réservoir de réactif avec l'outil de perçage (3600), dans lequel le premier fluide est stocké dans le réservoir de réactif.

18. Procédé selon la revendication 17, dans lequel le joint d'étanchéité est un capuchon perçable.

19. Procédé la revendication 17, dans lequel le réservoir de réactif est choisi dans le groupe constitué par un flacon et un bac.
